# EUROPÄISCHE PATENTSCHRIFT

(11) **EP 0 652 882 B1**
(45) Veröffentlichungstag und Bekanntmachung des Hinweises auf die Patenterteilung: **23.08.2000**
(21) Anmeldenummer: 93917694.7
(22) Anmeldetag: 29.07.1993
(51) Int. Cl.: C07D 498/04, A01N 43/90

(54) **ANELLIERTE (OXA)HYDANTOINE UND IHRE VERWENDUNG ALS HERBIZIDE**
ANELLATED (OXA)HYDANTOINES AND THEIR USE AS HERBICIDES
(OXA)HYDANTOINES ANNELEES ET LEUR UTILISATION COMME HERBICIDES

(30) Priorität: 30.07.1992 DE 4225167
(43) Veröffentlichungstag der Anmeldung: 17.05.1995
(73) Patentinhaber: Degussa-Hüls Aktiengesellschaft, 60287 Frankfurt am Main (DE)
(72) Erfinder: SCHÄFER, Matthias, D-63808 Haibach (DE); BAIER, Helmut, D-61273 Wehrheim (DE); DRAUZ, Karlheinz, D-63579 Freigericht (DE); KRIMMER, Hans-Peter 3-3-15 Den-en-chofu, Tokyo 145 (JP); LANDMANN, Sabine, D-63571 Gelnhausen (DE)
(86) Internationale Anmeldenummer: EP9302026
(87) Internationale Veröffentlichungsnummer: WO9403458

(56) Entgegenhaltungen:
- EP-A- 0 272 594
- JOURNAL OF ORGANIC CHEMISTRY Bd. 44, Nr. 26 , 1979 , EASTON US Seiten 4877 - 4880 R. RICHTER ET AL '5-Aryl-7-(N-arylcarbamoy l)-4,6-dioxo-2,3,3a,4,5,6-hexahydrooxa(thi a)zolo(2,3-c)pyrimidines and 3-(N-arylcarb amoyl)-2,4-dihydroxyquinolines from 2-methyloxa(thia)zoline and aryl isocyanates'

## Beschreibung

Die vorliegende Erfindung betrifft neuartige anellierte (Oxa)-hydantoine der Formel I 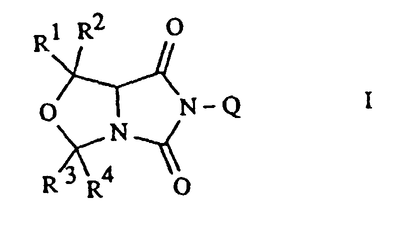 in welcher R¹ bis R⁴ sowie Q die in der Beschreibung genannte Bedeutung haben; eine Methode zu ihrer Herstellung; und ihre Verwendung als Herbizide.

Wie schon mitgeteilt wurde, können bestimmte (Thia)-hydantoine (siehe EP-A2 EP-A2 0 290 902) oder heterozyklische Imide (siehe EP-A1 272 594, EP-B1 0 070 389) als Herbizide verwendet werden.

Nun wurden überraschenderweise neuartige bizyklische Imide gefunden, die deutlich bessere herbizide Wirkung und ausgezeichnete Selektivität besitzen.

Die vorliegende Erfindung umfaßt daher Verbindungen der Formel I 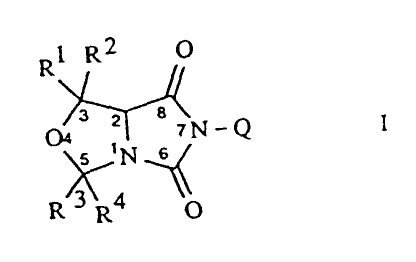 in welcher R¹ und R², unabhängig voneinander, für Wasserstoff oder eine Gruppe der Reihe (C₁ - C₄)Alkyl, (C₁ - C₂)Haloalkyl oder Phenyl, das gegebenenfalls fluorsubstituiert ist, stehen,
R³ und R⁴, unabhängig voneinander, für Wasserstoff, (C₁ - C₄)Alkyl, Phenyl, beide gegebenenfalls fluorsubstituiert, und/oder chlor, brom-, oder methylsubstituiert, (C₁ - C₄)Alkoxy stehen; oder auch zusammen einen carbocyclischen Ring bilden, der gegebenenfalls (C₁ - C₄)alkylsubstituiert sein kann,
Q für einen der Reste Q₁ - Q₇ steht 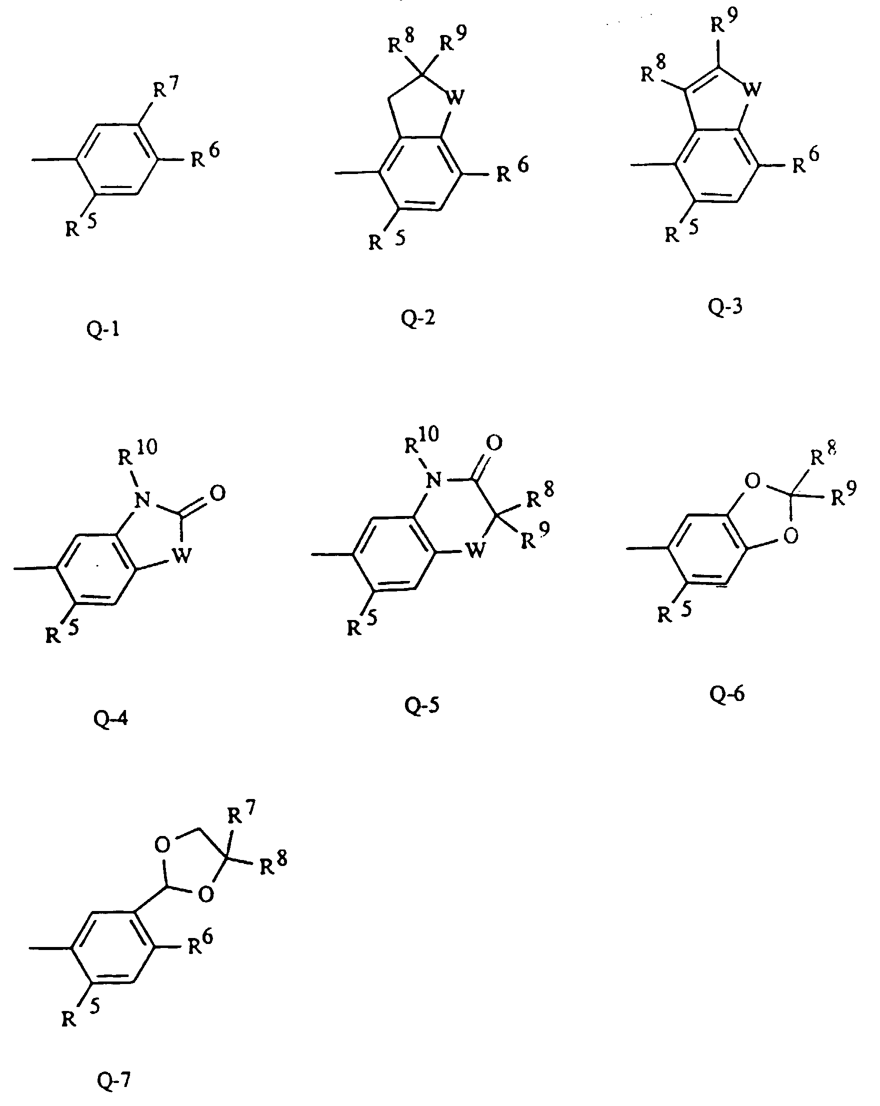 worin
   - W: für O oder S steht,
   - R⁵: für Wasserstoff oder Halogen steht,
   - R⁶: für (C₁ - C₂)Alkyl, (C₁ - C₂)Haloakyl, OCH₃, SCH₃, OCHF₂, Halogen, CN oder NO₂ steht,
   - R⁷: für (C₁ - C₈)Alkyl, (C₁ - C₈)Haloalkyl, Halogen, OR¹¹, S(O)ₙR¹¹, COR¹¹, CO₂R¹¹, C(O)SR¹¹, C(O)NR¹²R¹³, CHO, CH=CHCO₂R¹¹, CO₂N=CR¹⁴R¹⁵, NO₂, CN, NHSO₂R¹⁶ oder NHSO₂NHR¹⁶ steht,
   - R⁸: für Wasserstoff, (C₁ - C₃)Alkyl, (C₁ - C₃)Haloalkyl oder Halogen steht,
   - R⁹: für Wasserstoff, (C₁ - C₃)Alkyl, (C₁ - C₃)Haloalkyl oder Halogen steht; oder wenn Q = Q-2 oder Q-6 ist, können R⁸ und R⁹ zusammen mit dem Kohlenstoffatom, an das sie gebunden sind, C=O sein;
   - R¹⁰: für (C₁ - C₆)Alkyl, (C₁ - C₆)Haloalkyl, (C₂ - C₆)Alkoxyalkyl, (C₃ - C₆)Alkenyl oder (C₃ - C₆)Alkinyl steht,
   - R¹¹: für (C₁ - C₈)Alkyl, (C₃ - C₈)Cycloalkyl, (C₃ - C₈)Alkenyl, (C₃ - C₈)Alkinyl, (C₁ - C₈)Haloalkyl, (C₂ - C₈)Alkoxyalkyl, (C₂ - C₈)Alkylthioalkyl, (C₂ - C₈)Alkylsulfinylalkyl, (C₂ - C₈) Alkylsulfonylalkyl, (C₃ - C₈)Alkoxyalkoxyalkyl, (C₄ - C₈)Cycloalkylalkyl, (C₂ - C₄)Carboxyalkyl, (C₃ - C₈)Alkoxycarbonylalkyl, (C₆ - C₈)Alkenyloxycarbonylalkyl, (C₆ - C₈)Alkinyloxycarbonylalkyl, (C₄ - C₈)Alkenoxyalkyl, (C₆ - C₈)Cycloalkoxyalkyl, (C₄ - C₈)Alkinyloxyalkyl, (C₃ - C₈)Haloalkoxyalkyl, (C₄ - C₈)Haloalkenyloxyalkyl, (C₄ - C₈)Haloalkinyloxyalkyl, (C₆ - C₈)Cycloalkylthioalkyl, (C₄ - C₈)Alkenylthioalkyl, (C₄ - C₈)Alkinylthioalkyl, (C₁ - C₄)Alkyl substituiert mit Phenoxy oder Benzyloxy, die beide gegebenenfalls Halogen, (C₁ - C₃)Alkyl oder (C₁ - C₃)Haloalkylsubstituiert sein können; (C₄ - C₈)Trialkylsilylalkyl, (C₃ -C₈)Cyanoalkyl, (C₃ - C₈)Halocycloalkyl, (C₃ - C₈)Haloalkenyl, (C₅ - C₈)Alkoxyalkenyl, (C₅ - C₈)Haloalkoxyalkenyl, (C₅ - C₈)Alkylthioalkenyl, (C₃ - C₈)Haloalkinyl, (C₅ - C₈)Alkoxyalkinyl, (C₅ - C₈)Haloalkoxyalkinyl, (C₅ - C₈)Alkylthioalkinyl, (C₂ - C₈)Alkylcarbonyl, Benzyl gegebenenfalls substituiert mit Halogen, (C₁ - C₃)Alkyl oder (C₁ - C₃)Haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, Phenyl oder Pyridyl, beide gegebenenfalls substituiert mit Halogen, (C₁ - C₃)Alkyl, (C₁ - C₃)Haloalkyl oder (C₁ - C₄)Alkoxy, steht,
   - R¹² und R¹⁴,: unabhängig voneinander, für Wasserstoff oder (C₁ - C₄)Alkyl stehen,
   - R¹³ und R¹⁵,: unabhängig voneinander, für (C₁ - C₄)Alkyl, Phenyl, gegebenenfalls substituiert mit Halogen, (C₁ - C₃)Alkyl, (C₁ - C₃)Haloalkyl oder (C₁ - C₄)Alkoxy, stehen, oder
   - R¹² und R¹³: als -(CH₂)₅-, -(CH₂)₄- oder -CH₂CH₂OCH₂CH₂- zu Ringen kombiniert sein können, wobei ein oder mehrere H-Atome in jedem Ring gegebenenfalls durch (C₁ - C₃)Alkyl, Phenyl- oder Benzyl substituiert sein können;
   - R¹⁴ und R¹⁵: zusammen mit dem Kohlenstoffatom, an den sie gebunden sind, eine (C₃ - C₈)Cycloalkylgruppe bilden können,
   - R¹⁶: für (C₁ - C₄)Alkyl oder (C₁ - C₄)Haloalkyl steht,
   - R¹⁷: für Wasserstoff oder (C₁ - C₃)Alkyl steht,
   - R¹⁸: für (C₁ - C₆)Alkyl, (C₃ - C₆)Alkenyl oder (C₃ - C₆)Alkinyl steht, und
   - n: für 0, 1, 2 steht.

In den oben angegebenen Definitionen beinhaltet der Ausdruck "Alkyl", alleine oder in der Verbindungsbezeichnung wie "Alkylthio" oder "Haloalkyl" eine geradlinige oder verzweigte Kette, z. B., Methyl, Ethyl, n-Propyl, Isopropyl oder die verschiedenen Butylisomere. Alkoxy beinhaltet Methoxy, Ethoxy, n-Propyloxy, Isopropyloxy- und die verschiedenen Butoxyisomere. Alkenyl beinhaltet geradlinige oder verzweigte Alkene, z. B., 1-Propenyl, 2-Propenyl, 3-Propenyl und die verschiedenen Butenylisomere. Cycloalkyl beinhaltet Cyclopropyl, Cyclobutyl, Cyclopentyl und Cyclohexyl. Die Bezeichnung "Halogen", alleine oder in der Verbindungsbezeichnung wie "Haloalkyl" bedeutet Fluor, Chlor, Brom oder Jod. Desweiteren, wenn "Haloalkyl" in der Verbindungsbezeichnung benutzt wird, so kann "Alkyl" teilweise oder komplett mit Halogenatomen substituiert sein, die ihrerseits gleich oder verschieden sein können. Zu Beispielen für Haloalkyl gehören CH₂CH₂F, CF₂CF₃ und CH₂CHFCl.

Bevorzugt sind folgende Reste, in welchen
- R¹ und R²,: unabhängig voneinander, für Wasserstoff oder eine Gruppe der Reihe (C₁ - C₄)Alkyl, (C₁ - C₂)Haloalkyl oder Phenyl, das gegebenenfalls fluorsubstituiert ist, stehen,
- R³ und R⁴,: unabhängig voneinander, für Wasserstoff, (C₁ - C₃) Alkyl, Phenyl, gegebenenfalls fluorsubstituiert und/oder chlor-, brom, methylsubstituiert, (C₁ - C₂)Alkoxy stehen; oder zusammen einen carbocyclischen Ring bilden, der gegebenenfalls (C₁ - C₂)alkylsubstituiert sein kann,
- Q: bedeutet 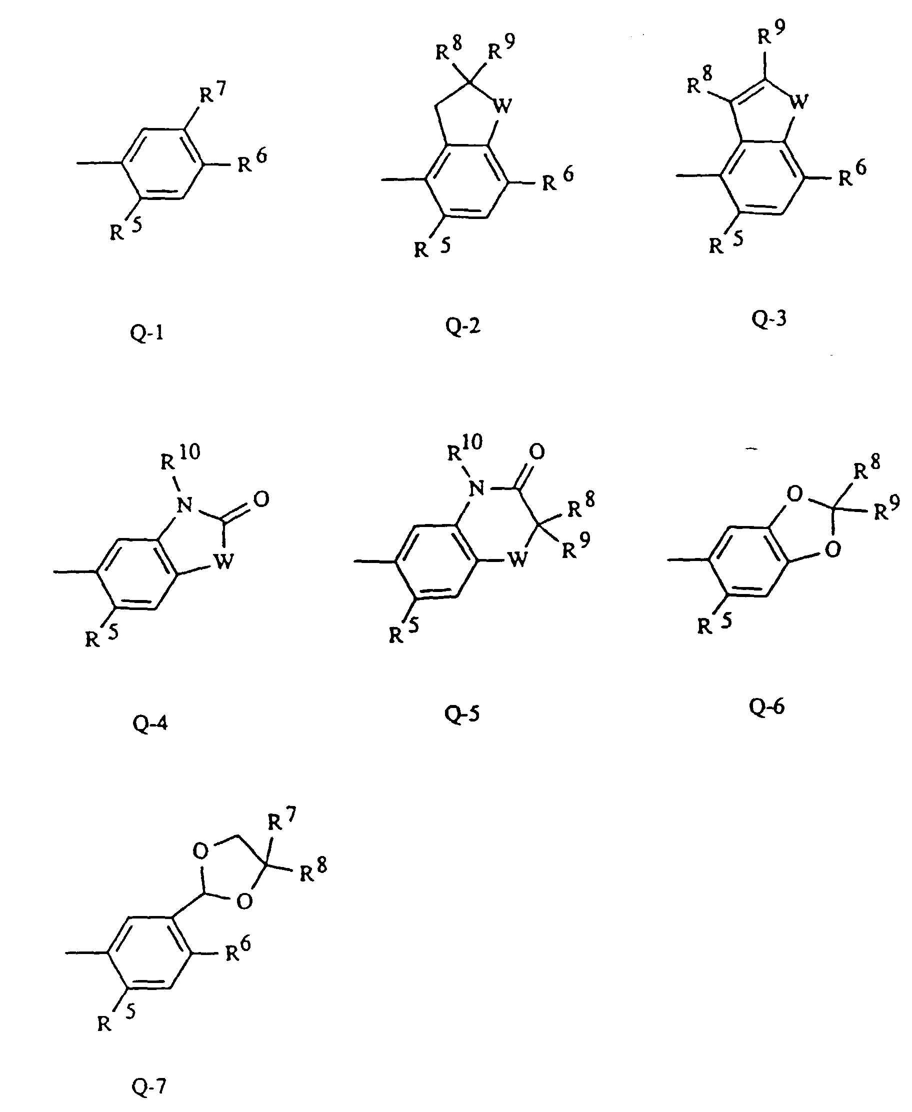 worin
- W: für O oder S steht,
- n: für 0, 1, 2 steht,
- R⁵: für Wasserstoff oder Halogen steht,
- R⁶: für Halogen oder CN steht,
- R⁷: für (C₁ - C₄)Alkyl, (C₁ - C₄)Haloalkyl, Halogen, OR¹¹, S(O)ₙR¹¹, COR¹¹, CO₂R¹¹, C(O)SR¹¹, C(O)NR¹²R¹³, CH=CHCO₂R¹¹, CO₂N=CR¹⁴R¹⁵, NHSO₂R¹⁶ oder NHSO₂NHR¹⁶ steht,
- R⁸: für Wasserstoff, (C₁ - C₃)Alkyl oder (C₁ - C₃)Haloalkyl steht,
- R⁹: für Wasserstoff, (C₁ - C₃)Alkyl oder (C₁ - C₃)Haloalkyl steht; oder wenn Q = Q-2 oder Q-6 ist, können R⁸ and R⁹ zusammen mit dem Kohlenwasserstoffatom, an das sie gebunden sind, C=O sein;
- R¹⁰: für (C₁ - C₄)Alkyl, (C₁ - C₄)Haloalkyl, (C₂ - C₄)Alkoxyalkyl, (C₃ - C₆)Alkenyl oder (C₃ - C₆)Alkinyl steht,
- R¹¹: für (C₁ - C₄)Alkyl, (C₃ - C₆)Cycloalkyl, (C₃ - C₆)Alkenyl, (C₃ - C₆)Alkinyl, (C₁ - C₄)Haloalkyl, (C₂ - C₄)Alkoxyalkyl, (C₂ - C₄)Alkylthioalkyl, (C₂ - C₄)Alkylsulfinylalkyl, (C₂ - C₄) Alkylsulfonylalkyl, (C₃ - C₆)Alkoxyalkoxyalkyl, (C₄ - C₈) Cycloalkylalkyl, (C₂ - C₄)Carboxyalkyl, (C₃ - C₆)Alkoxycarbonylalkyl, (C₆ - C₈)Alkenyloxycarbonylalkyl, (C₆ - C₈)Alkinyloxycarbonylalkyl, (C₄ - C₆)Alkenoxyalkyl, (C₆ - C₈)Cycloalkoxyalkyl, (C₄ - C₆)Alkinyloxyalkyl, (C₃ - C₆)Haloalkoxyalkyl, (C₄ - C₈)Haloalkenyloxyalkyl, (C₄ - C₆)Haloalkinyloxyalkyl, (C₆ - C₈)Cycloalkylthioalkyl, (C₄ - C₆)Alkenylthioalkyl, (C₄ - C₆)Alkinylthioalkyl, (C₁ - C₂)Alkyl substituiert mit Phenoxy oder Benzyloxy, die beide gegebenenfalls mit Halogen, (C₁ - C₃)Alkyl oder (C₁ - C₃)Haloalkyl substituiert sein können; (C₄ - C₈)Trialkylsilylalkyl, (C₃ - C₄)Cyanoalkyl, (C₃ - C₆)Halocycloalkyl, (C₃ - C₆)Haloalkenyl, (C₅ - C₆)Haloalkoxyalkenyl, (C₅ - C₆)Alkylthioalkenyl, (C₃ - C₆)Haloalkinyl, (C₅ - C₆)Alkoxyalkinyl, (C₅ - C₆)Haloalkoxyalkinyl, (C₅ - C₆)Alkylthioalkinyl, (C₂ - C₄)Alkylcarbonyl, Benzyl gegebenenfalls substituiert mit Halogen, (C₁ - C₂)Alkyl oder (C₁ - C₂)Haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, Phenyl oder Pyridyl, beide gegebenenfalls substituiert mit Halogen, (C₁ - C₃)Alkyl, (C₁ - C₃)Haloalkyl oder (C₁ - C₄)Alkoxy, steht,
- R¹² und R¹⁴,: unabhängig voneinander, für Wasserstoff oder (C₁ - C₂)Alkyl stehen,
- R¹³ und R¹⁵,: unabhängig voneinander, für (C₁ - C₂)Alkyl, Phenyl gegebenenfalls substituiert mit Halogen, (C₁ - C₂)Alkyl, (C₁ - C₂)Haloalkyl oder (C₁ - C₂)Alkoxy, stehen, oder
- R¹² und R¹³: als -(CH₂)₅-, -(CH₂)₄- oder -CH₂CH₂OCH₂CH₂- zu Ringen kombiniert sein können, wobei ein oder mehrere H-Atome in jedem Ring gegebenenfalls durch (C₁ - C₂)Alkyl-, Phenyl- oder Benzyl subsituiert sein können,
- R¹⁴ und R¹⁵: zusammen mit dem Kohlenstoff, an den sie gebunden sind, eine (C₃ - C₆)Cycloalkylgruppe bilden können,
- R¹⁶: für (C₁ - C₄)Alkyl oder (C₁ - C₄)Haloalkyl steht,
- R¹⁷: für Wasserstoff oder (C₁ - C₃)Alkyl steht,
- R¹⁸: für (C₁ - C₄)Alkyl, (C₃ - C₄)Alkenyl oder (C₃ - C₄)Alkinyl steht.

Bevorzugt sind folgende Reste, in welchen
- R¹ und R²,: unabhängig voneinander, für Wasserstoff oder eine Gruppe der Reihe (C₁ - C₃)Alkyl, (C₁ - C₂)Haloalkyl oder Phenyl, das gegebenenfalls floursubstituiert ist, steht,
- R³ und R⁴,: unabhängig voneinander, für Wasserstoff, (C₁ - C₃) Alkyl stehen, oder auch zusammen einen 5 - 6 gliedrigen carbocyclischen Ring bilden, der gegebenenfalls (C₁ - C₄)alkylsubstituiert sein kann,
- Q: bedeutet 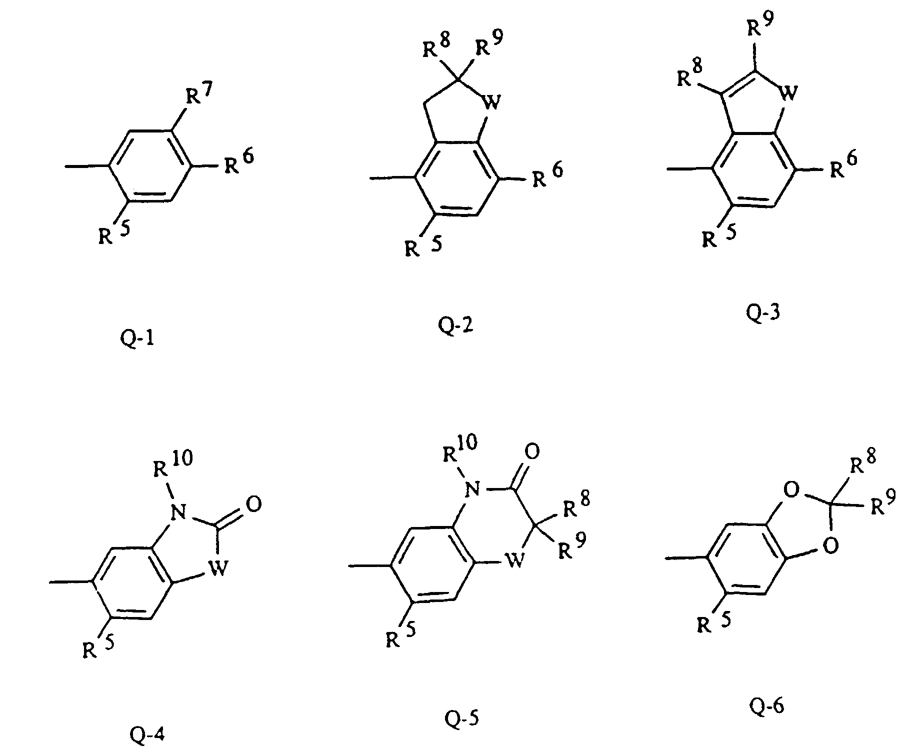 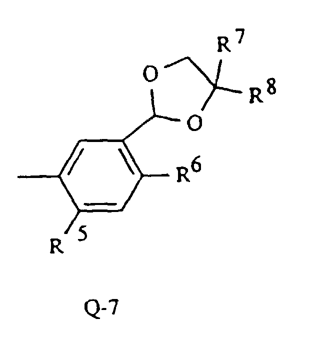 worin
- W: für O oder S steht,
- n: für 0, 1 oder 2 steht,
- R⁵: für Wasserstoff, Fluor oder Chlor steht,
- R⁶: für Chlor, Brom oder Cyan steht,
- R⁷: für Wasserstoff, OR¹¹ oder CO₂R¹¹ steht,
- R⁸ und R⁹,: unabhängig voneinander, für Wasserstoff, (C₁ - C₂)Alkyl oder (C₁ - C₂)Haloalkyl stehen,
- R¹⁰: für (C₁ - C₂)Alkyl, (C₁ - C₂)Haloalkyl, (C₃ - C₄)Alkenyl, oder (C₃ - C₄)Alkinyl stehen,
- R¹¹: für (C₁ - C₄)Alkyl, (C₃ - C₆)Cycloalkyl, (C₃ - C₆)Alkenyl, (C₃ - C₆)Alkinyl, (C₁ - C₄)Haloalkyl, (C₂ - C₄)Alkoxyalkyl, (C₂ - C₄)Alkylthioalkyl, (C₂ - C₄)Alkylsulfinylalkyl, (C₂ - C₄)Alkylsulfonylalkyl, (C₃ - C₆)Alkoxyalkoxyalkyl, (C₄ - C₈)Cycloalkylalkyl, (C₂ - C₄)Carboxyalkyl, (C₃ - C₆)Alkoxycarbonylalkyl, (C₆ - C₈)Alkenyloxycarbonylalkyl, (C₆ - C₈)Alkinyloxycarbonylalkyl, (C₆ - C₈)Cycloalkoxyalkyl, (C₄ - C₆)Alkenyloxyalkyl, (C₄ - C₆)Alkinyloxyalkyl, (C₃ - C₆)Haloalkoxyalkyl, (C₄ - C₈)Haloalkenoxyalkyl, (C₄ - C₆)Haloalkinyloxyalkyl, (C₆ - C₈)Cycloalkylthioalkyl, (C₄ - C₆)Alkenylthioalkyl, (C₄ - C₆)Alkinylthioalkyl, (C₁ - C₂)Alkyl substituiert mit Phenoxy oder Benzyloxy, die beide gegebenenfalls substituiert sind mit Halogen, (C₁ - C₃)Alkyl oder (C₁ - C₃)Haloalkyl; (C₄ - C₈)Trialkylsilylalkyl, (C₃ - C₄)Cyanoalkyl, (C₃ - C₆)Halocycloalkyl, (C₃ - C₆)Haloalkenyl, (C₅ - C₆)Alkoxyalkenyl, (C₅ - C₆)Haloalkoxyalkenyl, (C₅ - C₆)Alkylthioalkenyl, (C₃ - C₆)Haloalkinyl, (C₅ - C₆)Alkoxyalkinyl, (C₅ - C₆)Haloalkoxyalkinyl, (C₅ - C₆)Alkylthioalkinyl, (C₂ - C₄)Alkylcarbonyl, Benzyl gegebenenfalls substituiert mit Halogen, (C₁ - C₂)Alkyl oder (C₁ - C₂)Haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, Phenyl oder Pyridyl, beide gegebenenfalls substituiert mit Fluor, Chlor, Brom, (C₁ - C₂)Haloalkyl oder (C₁ - C₂)Alkoxy, steht,
- R¹²: für Wasserstoff oder (C₁ - C₂)Alkyl steht,
- R¹³: für (C₁ - C₂)Alkyl, Phenyl gegebenenfalls substituiert mit Fluor, Chlor, Brom, (C₁ - C₂)Alkyl, (C₁ - C₂)Haloalkyl oder (C₁ - C₂)Alkoxy, steht, oder
- R¹² und R¹³: können als -(CH₂)₅-, -(CH₂)₄- oder -CH₂CH₂OCH₂CH₂- zu Ringen kombiniert sein, wobei ein oder mehrere H-Atome in jedem Ring gegebenenfalls durch (C₁ - C₂)Alkyl substituiert sein können,
- R¹⁷: für Wasserstoff oder (C₁ - C₂)Alkyl steht,
- R¹⁸: für (C₁ - C₂)Alkyl, (C₃ - C₄)Alkenyl oder (C₃ - C₄)Alkinyl steht.

Bevorzugt sind folgende Reste, in welchen
- R¹ und R²,: unabhängig voneinander, für Wasserstoff, (C₁ - C₃)Alkyl, (C₁ - C₂)Haloalkyl oder Phenyl stehen,
- R³ und R⁴,: unabhängig voneinander, für Wasserstoff oder (C₁ - C₃)Alkyl stehen oder auch zusammen einen 5 - 6 gliedrigen carbocyclischen Ring bilden können,
- Q: bedeutet 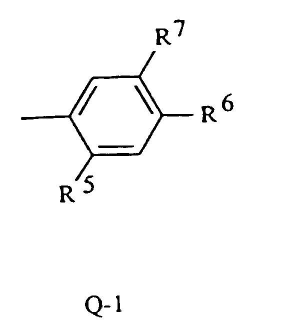 worin
- R⁵: für Fluor oder Chlor steht,
- R⁶: für Chlor steht,
- R⁷: für OR¹¹ oder CO₂R¹¹ steht,
- R¹¹: für (C₁ - C₄)Alkyl, (C₃ - C₆)Cycloalkyl, (C₃ - C₆)Alkenyl, (C₃ - C₄)Alkinyl, (C₁ - C₃)Haloalkyl, (C₂ - C₄)Alkoxyalkyl, (C₃ - C₆)Alkoxycarbonylalkyl, (C₆ - C₈)Alkenyloxycarbonylalkyl oder (C₆ - C₈)Alkinyloxycarbonyl steht.

Die Erfindung bezieht sich sowohl auf die möglichen einzelnen Stereoisomeren der Formel I als auch auf Gemische der Isomeren. Die Stereoisomeren der 2R,3S-Konfiguration sind gegenüber anderen bevorzugt.

Die neuartigen anellierten (Oxa)-hydantoine der allgemeinen Formel I erhält man nach der vorliegenden Erfindung nach einer allgemeinen Methode A, wenn Arylisocyanate der allgemeinen Formel II

Q―N=C=O II

in welcher
Q die oben angegebene Bedeutung hat, und
Oxazolidincarboxylsäure(ester) der allgemeinen Formel III 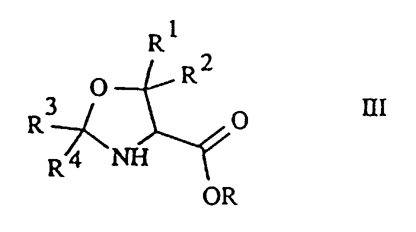 in welcher R¹, R², R³ und R⁴ (R³ = R⁴ 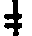 H) die oben angegebene Bedeutung haben und R = H, (C₁ -C₄)Alkyl oder Aktivester ist, entsprechend der Methode A gegebenenfalls in Gegenwart eines Säureakzeptors und gegebenenfalls in Gegenwart eines Verdünnungsmittels zur Reaktion gebracht werden. Eine ähnliche Vorgehensweise wird in J. Org. Chem. 1979, 26, 4877-80 beschrieben.

Ein weiterer Gegenstand der Erfindung ist eine Methode B zur Herstellung von Verbindungen der Formel I, die im folgenden erläutert wird und wobei R¹ bis R⁴ sowie Q die oben angegebene Bedeutung haben, wobei eine Verbindung der Formel III, in welcher R = H bedeutet und 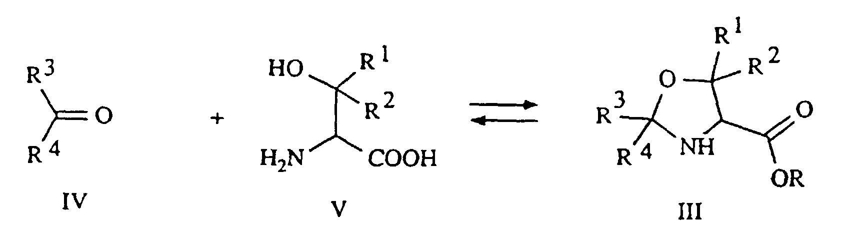 die unter Gleichgewichtsbedingungen aus Verbindungen der Formel IV und Verbindungen der Formel V oder deren Salzen, gegebenenfalls in Gegenwart eines Säureakzeptors und gegebenenfalls in Gegenwart eines Verdünnungsmittels hergestellt werden kann, mit einer Verbindung der Formel II, gegebenenfalls in Gegenwart eines Säureakzeptors und gegebenenfalls in Gegenwart eines Verdünnungsmittels, zur Reaktion gebracht wird, um Verbindungen der Formel VI zu erhalten, in der R = H, (C₁ - C₄)Alkyl oder ein Aktivester ist 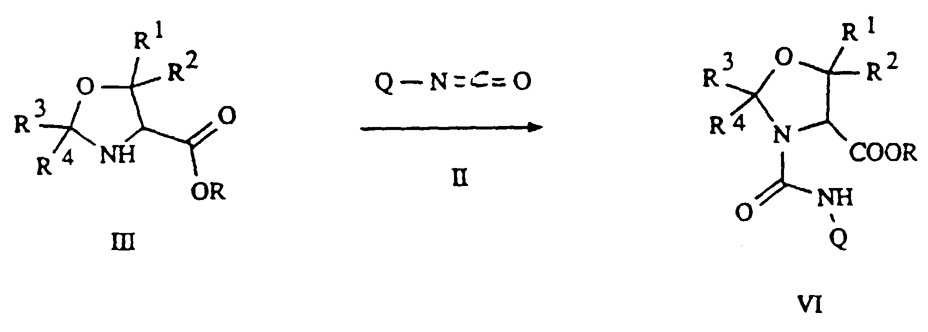 die dann unter Ringschluß in Verbindungen der Formel I umgewandelt werden.

Ein weiterer Gegenstand der Erfindung ist die Methode C zur Herstellung von Verbindungen der Formel I, die im folgenden erläutert wird und wobei R¹ bis R⁴ die oben angegebene Bedeutung haben. Eine Verbindung der Formel III, in welcher R = H oder (C₁ - C₄)Alkyl, wird dazu mit Phosgen oder einem Phosgenersatz zur Reaktion gebracht, wobei zunächst Verbindungen der Formel VII entstehen und diese Verbindungen dann mit Verbindungen der Formel VIII zur Reaktion gebracht werden, in welcher Q die oben angegebene Bedeutung besitzt, um Verbindungen der Formel VI 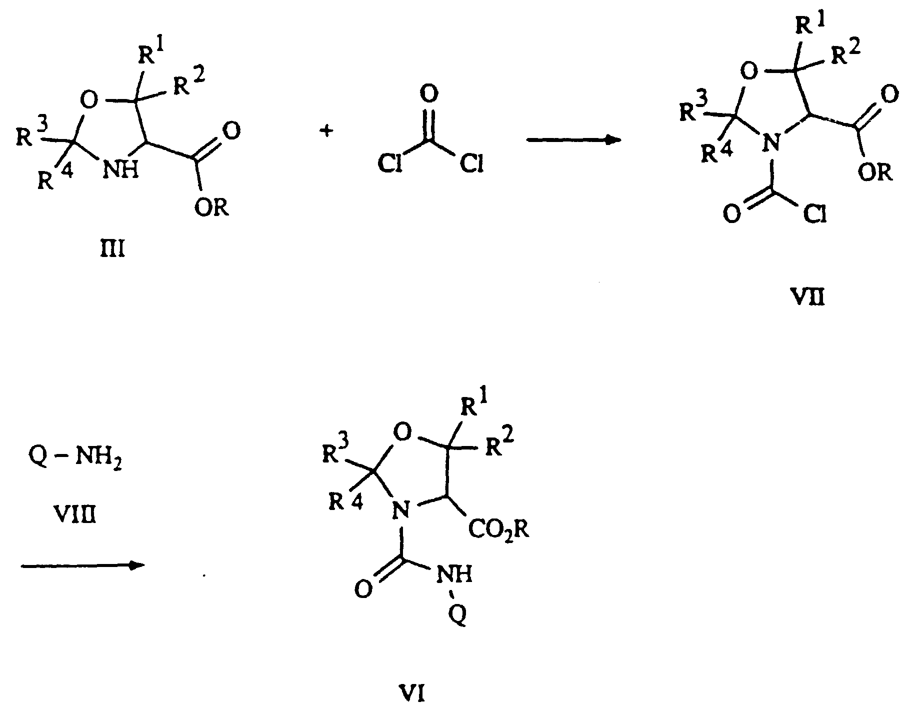 zu erhalten, die dann unter Ringschluß in Verbindungen der Formel I umgewandelt werden.

Ein weiterer Gegenstand der Erfindung ist die Methode D zur Herstellung von Verbindungen der Formel I, die im folgenden erläutert wird und wobei R¹ bis R⁴ sowie Q die oben angegebene Bedeutung haben, wobei eine Verbindung der Formel II mit einer Verbindung der Formel IX, gegebenenfalls in Gegenwart eines Säureakzeptors und gegebenenfalls in Gegenwart eines Verdünnungsmittels, zur Reaktion gebracht wird, um Verbindungen der Formel X zu erhalten, und die so erhaltenen Verbindungen X 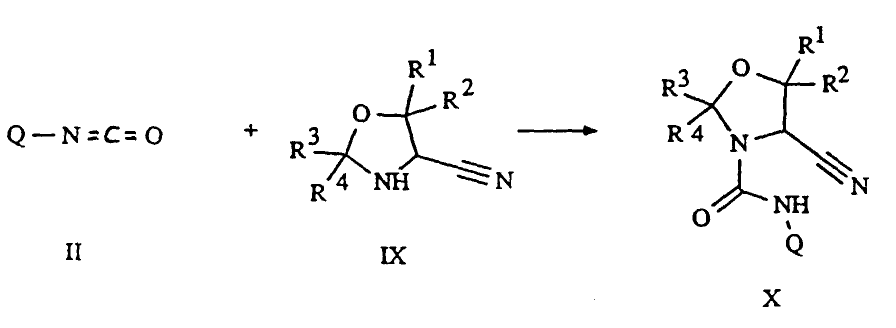 dann hydrolysiert und unter Ringschluß in Verbindungen der Formel I umgewandelt werden.

Ein weiterer Gegenstand der vorliegenden Erfindung ist eine Methode E zur Herstellung von Verbindungen der Formel I, indem man die Verbindungen der allgemeinen Formel XI 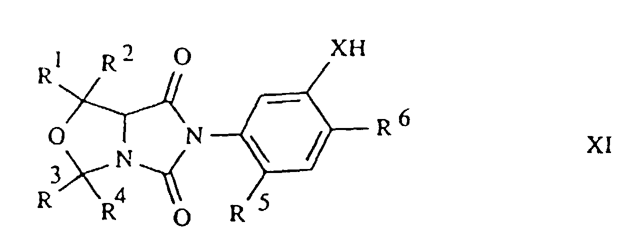 worin R¹ bis R⁶ die oben angegebene Bedeutung haben und X = 0, S oder NH bedeutet, mit einem Halogenid der Formeln XII, XIII oder XIV zur Reaktion gebracht werden, 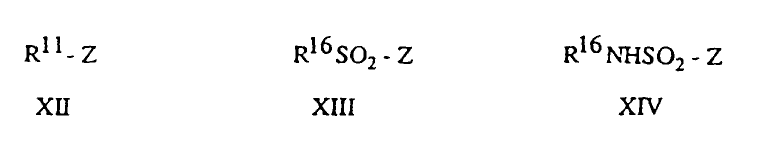 worin Z = Chlor, Brom- oder ein Jodatom bedeuten und R¹¹ und R¹⁶ die oben angegebene Bedeutung haben.

Schließlich ist gefunden worden, daß die neuartigen anellierten (Oxa)hydantoine der allgemeinen Formel I außergewöhnliche herbizide Eigenschaften besitzen.

Bei der Methode A wird die Reaktion im Falle von R = Alkyl in einem inerten organischen Lösemittel durchgeführt, z. B. in einem aromatischen Lösemittel wie Toluol, Chlorbenzol, einem halogenierten Kohlenwasserstoff wie Chloroform oder Methylenchlorid, einem Ether wie Diisopropylether, oder in Acetonitril oder Dimethylformamid, gegebenenfalls basenkatalysiert bei Temperaturen zwischen 20 und 120°C. Als Basen werden vorzugsweise organische Basen benutzt, z. B. organische Amine wie Triethylamin oder auch Pyridin (siehe EP-A1 0 272 594). Varianten der allgemeinen Methode A werden im EP-A2 0 070 389 beschrieben.

Bei der Methode B, die sich besonders im Falle von R³ und R⁴ = H bewährt hat, läuft die Reaktion in Wasser als Lösemittel oder, vorzugsweise, im Zweiphasensystem Wasser/organisches Lösemittel ab. Besonders vorzuziehen ist die Arbeitsweise, bei welcher Verbindungen der Formeln IV und V, gegebenenfalls Salze von V, zusammen mit einer anorganischen Base, z. B. einem Alkali- oder Erdalkalimetallhydroxid, -carbonat oder -hydrogencarbonat wie Natriumhydroxid oder auch Kaliumcarbonat, oder einer organischen Base, z. B. einem organischen Amin wie Triethylamin zugegeben und dann mehrere Tage, vorzugsweise 3 bis 10 h, auf einer Temperatur zwischen -40 und +50°C, vorzugweise -10 bis +10°C, gehalten werden.

Der Verbindung der Formel III, die in dieser wäßrigen Lösung im Gleichgewicht entsteht, wird das Isocyanat der Formel II, gelöst In einem inerten organischen Lösemittel, z. B. Toluol, Chlorbenzol oder Chloroform, tropfenweise unter kräftigem Rühren zugegeben.

Der pH-Wert der wäßrigen Phase wird dann mit Säure, vorzugsweise einer anorganischen Säure wie wäßriger Salz- oder Schwefelsäure, auf 1 bis 3 eingestellt. Die so entstandenen Harnstoffderivate der Formel VI werden dann bei einer Temperatur zwischen 50 und 100°C oder, gegebenenfalls, durch Umwandlung in einen Ester (R = Alkyl oder Aktivester) nach bekannten Methoden zyklisiert (siehe Houben-Weyl, Methoden der Organischen Chemie, Band XXV/1 und XXV/2 (1974).

Die Verbindungen der Formel II sind bekannt oder können analog den bekannten Methoden hergestellt werden; siehe Houben-Weyl, Methoden der Organischen Chemie, Band VIII, s. 120 (1952); Houben-Weyl, Band IX, s. 875, 869 (1955); EP-B1 0 070 389; US-4,881,967; EP-A1 322 401; US 3,495,967; EP-A2 300 307; EP-A2 349 832.

Amine der Formel III sind bekannt und können für R³ = R⁴ 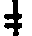 H analog bekannter Methoden hergestellt werden; siehe z. B. D. Seebach et al., Helv. Chim. Acta, Band 70, 1194 (1987).

Amine der allgemeinen Formel IX sind bekannt oder können entsprechend EP-A 3 073 569 oder auf analoge Weise entsprechend der dort beschriebenen Methode hergestellt werden.

Die Erfindung betrifft auch die Verwendung der Verbindungen der Formel I als Herbizide sowie herbizide Zusammensetzungen, die einen effektiven Gehalt an einer Verbindung der Formel I und einen Trägerstoff enthalten. Trägerstoffe sind vorteilhaft oberflächenaktive Stoffe, feste oder flüssige Verdünner.

Die Erfindung betrifft auch ein Verfahren zur Schadpflanzenkontrolle, bei dem auf die Schadpflanzen oder ihre Umgebung (vor oder nach dem Auflaufen) eine herbizid wirksame Menge einer Verbindung gemäß Formel I ausgebracht wird.

### Chemische Beispiele

### Beispiel 1:

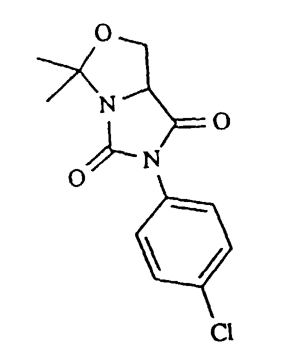

Ein Gemisch von 2,2-Dimethyloxazolidin-4-carboxylsäuremethylester (1,99 g, 0,01 mol), Triethylamin (50,0 mg, 0,5 mmol) und Toluol (30 ml) wird hergestellt und tropfenweise mit in 20 ml Toluol gelösten 4-Chlorphenylisocyanat (1,40 g, 0,009 mol) versetzt. Das Reaktionsgemisch wird bei 20°C 15 h lang gerührt und dann mit 10 %iger wäßriger Salzsäure (3 x 10 ml) und Wasser (3 x 10 ml) gewaschen, über Natriumsulfat getrocknet und filtriert. Nach Konzentrieren des Filtrats durch Eindampfen wird der Rückstand in Diethylether gelöst und aus Petrolether umgefällt.

Man erhält 2,28 g (81 % der theoretischen Menge) 7-(4-Chlorophenyl)-5,5-dimethyl-6,8-dioxo-4-oxa-1,7-diazabicyclo[3.3.0]octan mit Schmelzpunkt 97 - 98°C.

### Beispiel 2:

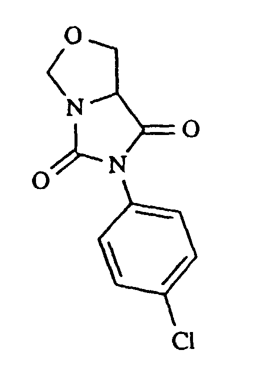

Ein Gemisch aus Serin (1,05 g, 0,01 mol), 37 %iger wäßriger Formaldehydlösung (2 g) und wäßriger Natriumhydroxidlösung (10 ml) wird 12 h auf 4°C gehalten, auf 0°C gekühlt und tropfenweise mit 4-Chlorophenylisocyanat (1,55 g) gelöst, in Chlorbenzol (5 ml), versetzt. Das Reaktionsgemisch wird dann 1/2 h bei 0 - 5°C und etwa 2 h bei Raumtemperatur gerührt, die wäßrige Phase wird mit Chloroform (3 x 10 ml) ausgeschüttelt und die gesammelten organischen Phasen werden verworfen.

Die wäßrige Phase wird dann mit 5 %iger wäßriger Salz säure auf pH 1 angesäuert und das Produkt durch Ausschütteln mit Ethylacetat (3 x 10 ml), in die organische Phase gebracht, über Natriumsulfat getrocknet und filtriert. Nach Konzentrieren des Filtrats durch Eindampfen wird der Rückstand in Acetonitril (25 ml) aufgenommen und mit N-Hydroxysuccinimid (1,15 g, 0,01 mol) zur Reaktion gebracht und dann bei 20°C tropfenweise mit N,N'-Dicyclohexylcarbodiimid (2,06 g, 0,01 mol), gelöst in Acetonitril (15 ml), versetzt. Das Reaktionsgemisch wird 12 h unter Rückfluß gerührt, dann filtriert und das Filtrat durch Eindampfen konzentriert. Der verbleibende Rückstand wird in etwas Aceton aufgenommen und mit Wasser erneut ausgefällt.

Das 7-(4-Chlorphenyl)-6,8-dioxo-4-oxa-1,7-diazabicyclo[3.3.0]octan mit Schmelzpunkt 74 - 76°C wird in einer Menge von 1,75 g (74 % der theoretischen Menge) erhalten.

Analog den Beispielen 1 und 2 und entsprechend der allgemeinen Bespreibung der Methoden A bis E nach der vorliegenden Erfindung können die in den nachstehenden Tabellen aufgeführten Verbindungen der allgemeinen Formel I hergestellt werden.

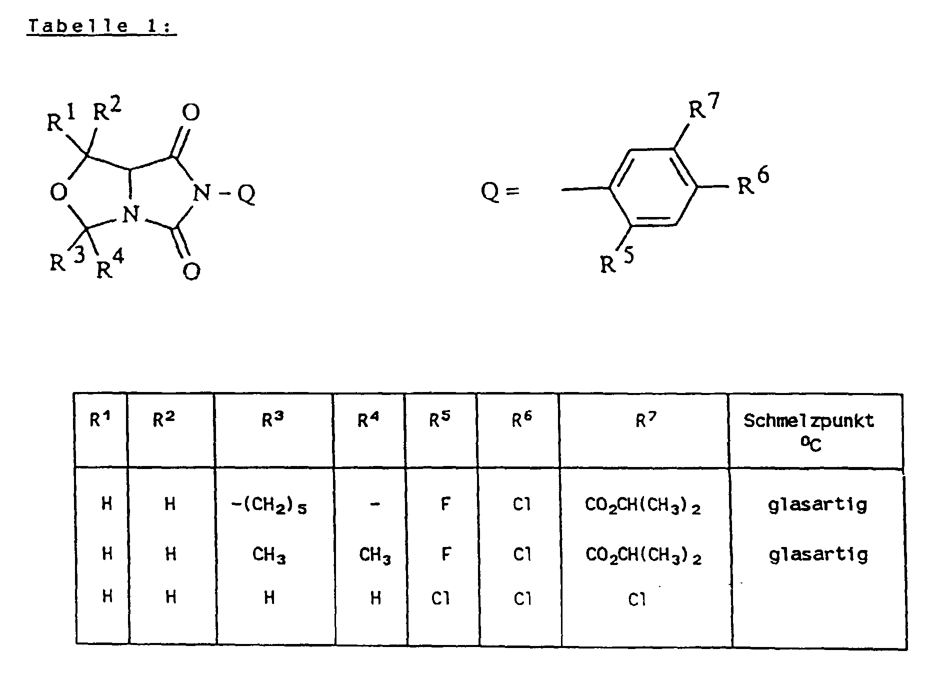

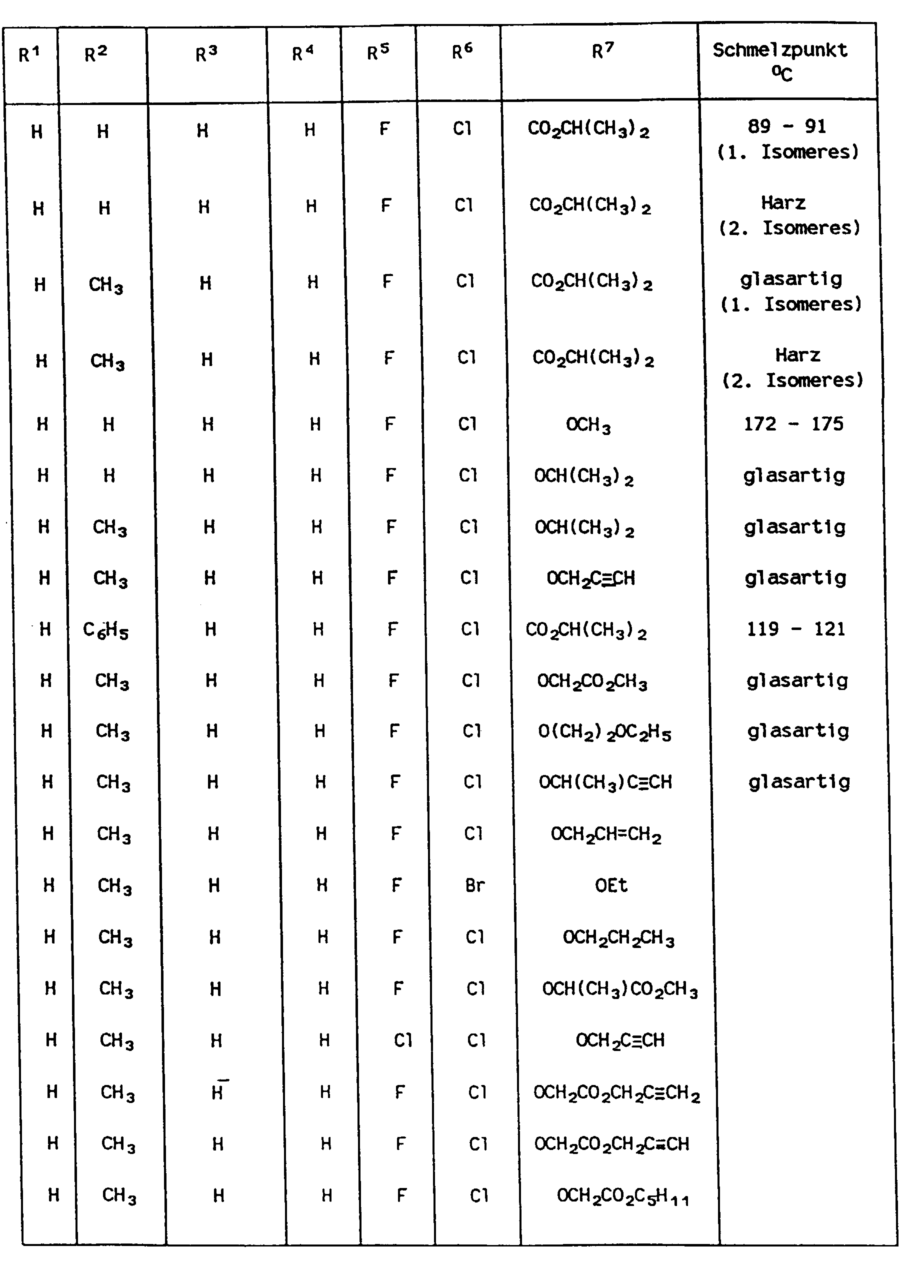

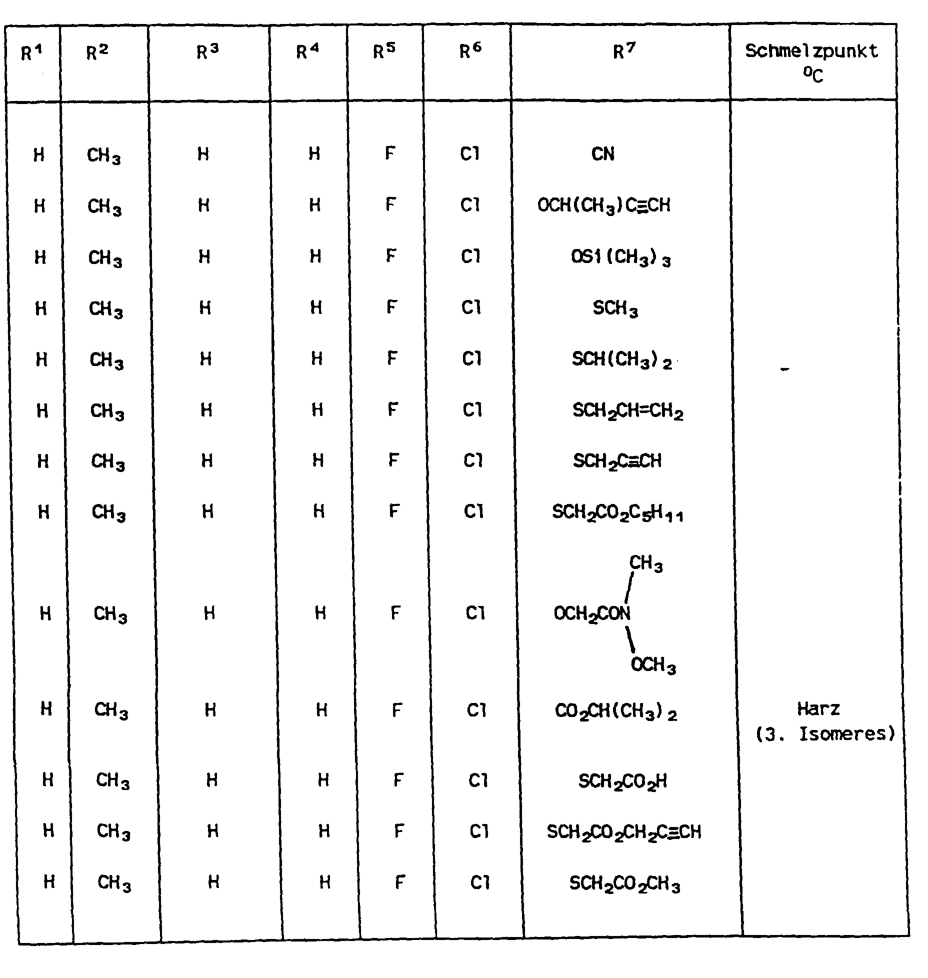

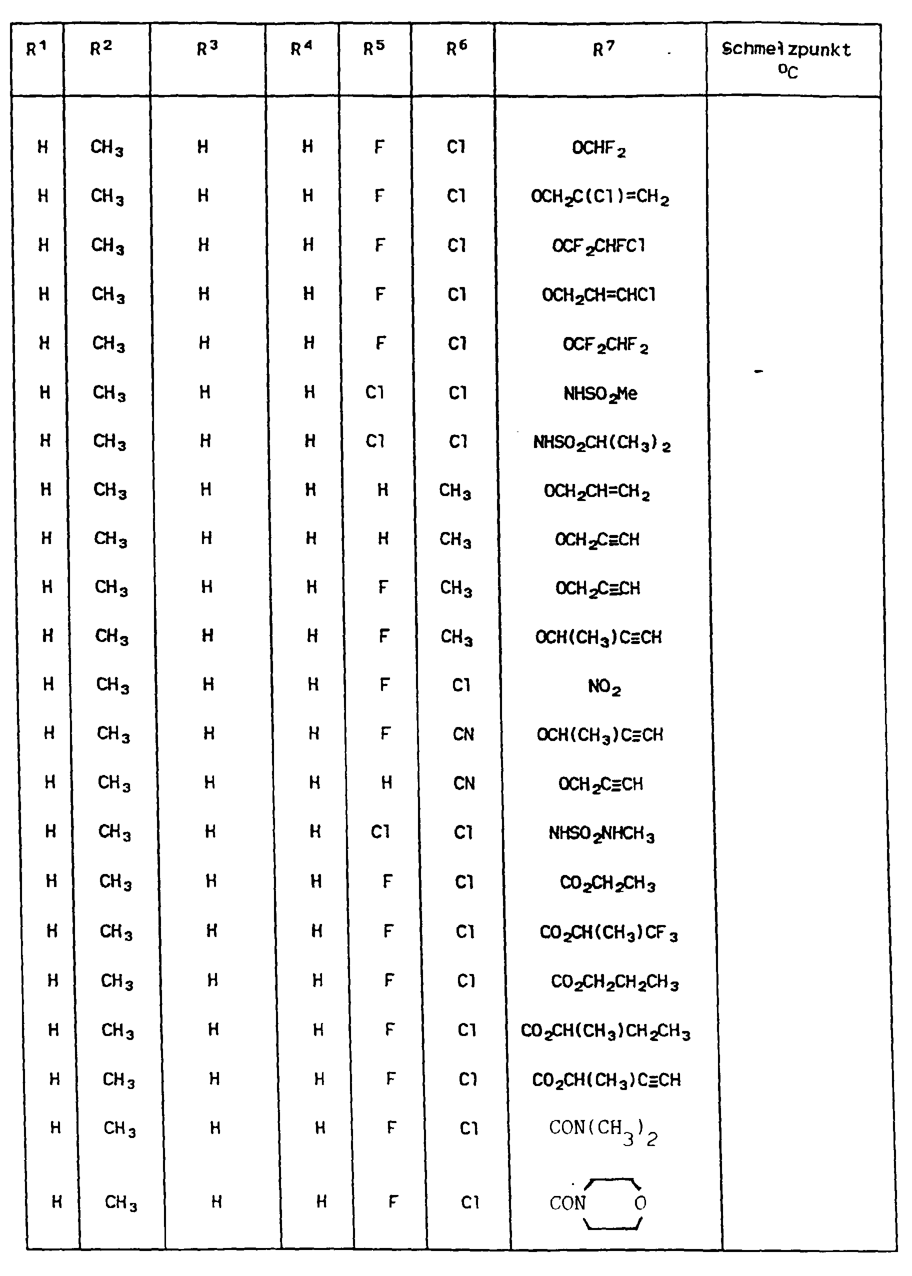

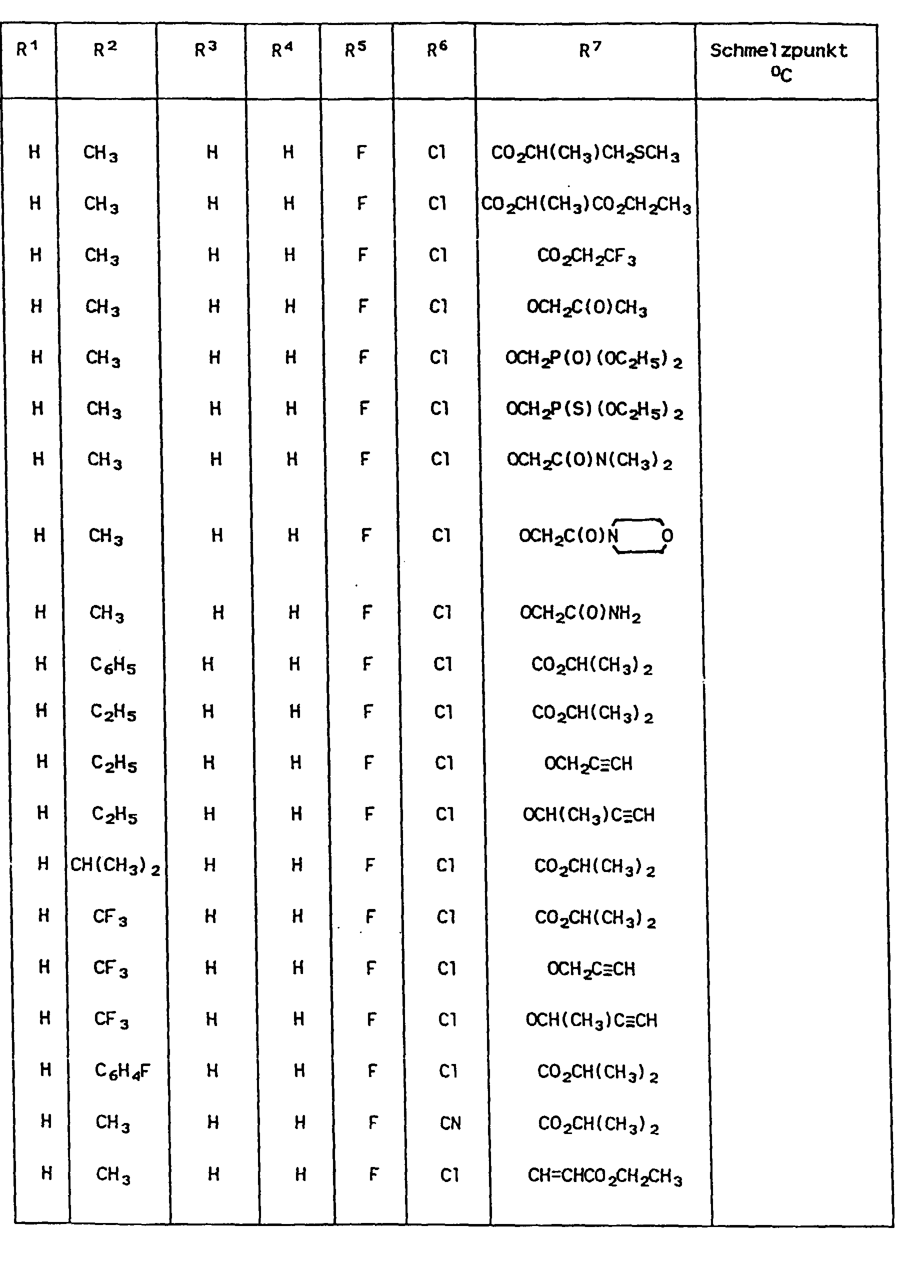

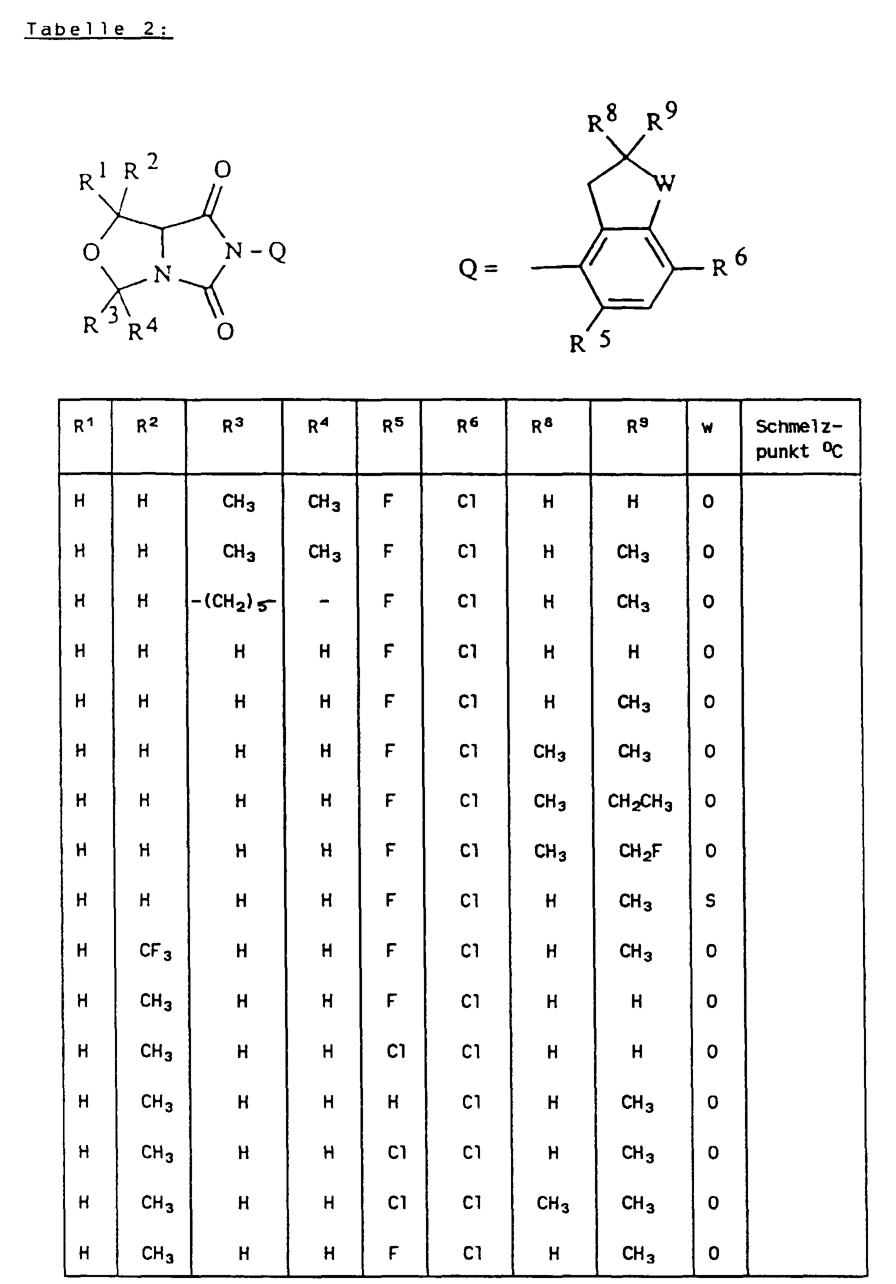

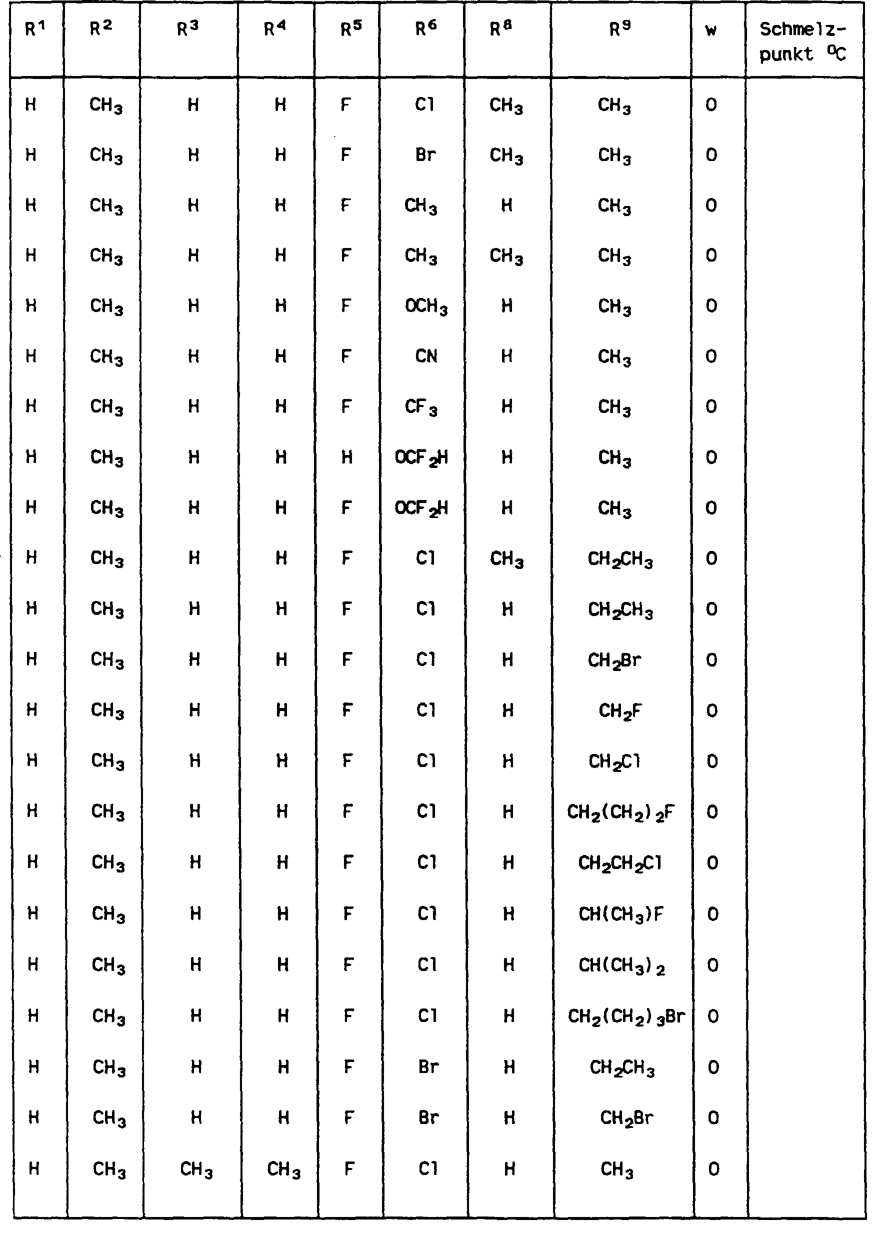

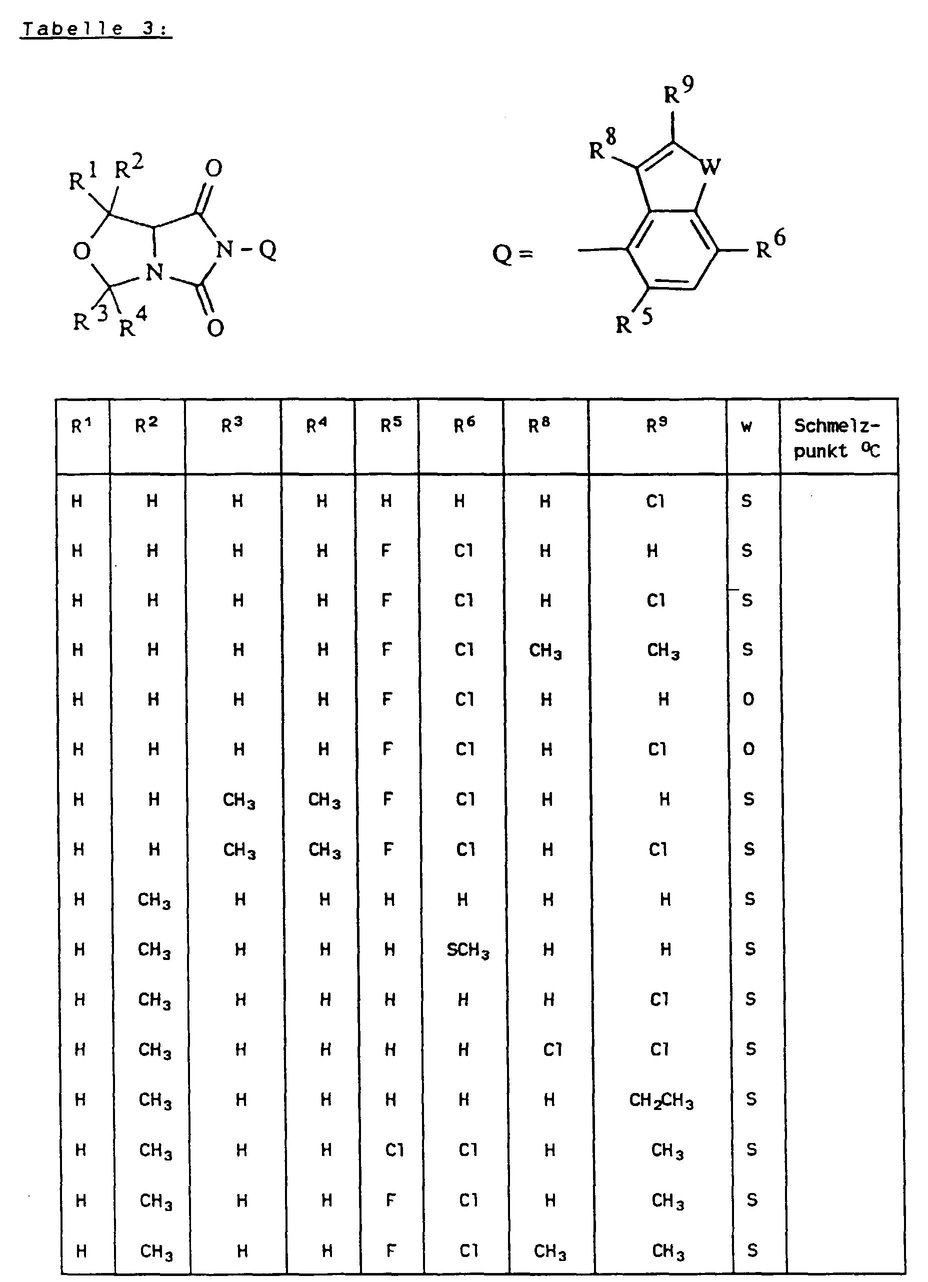

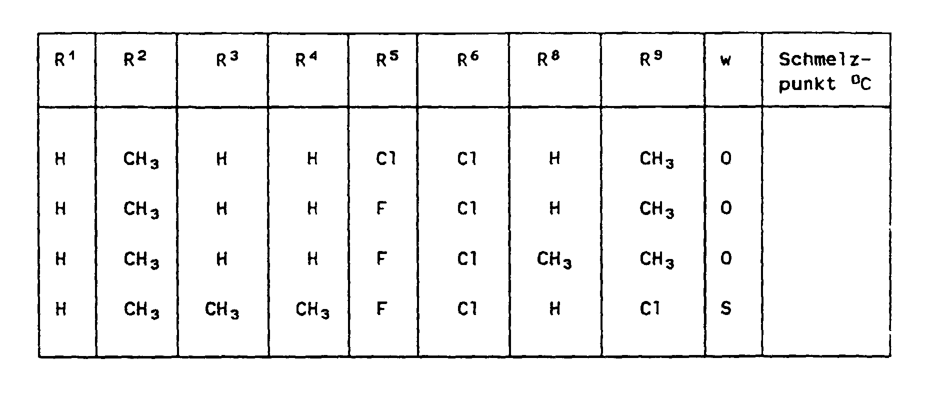

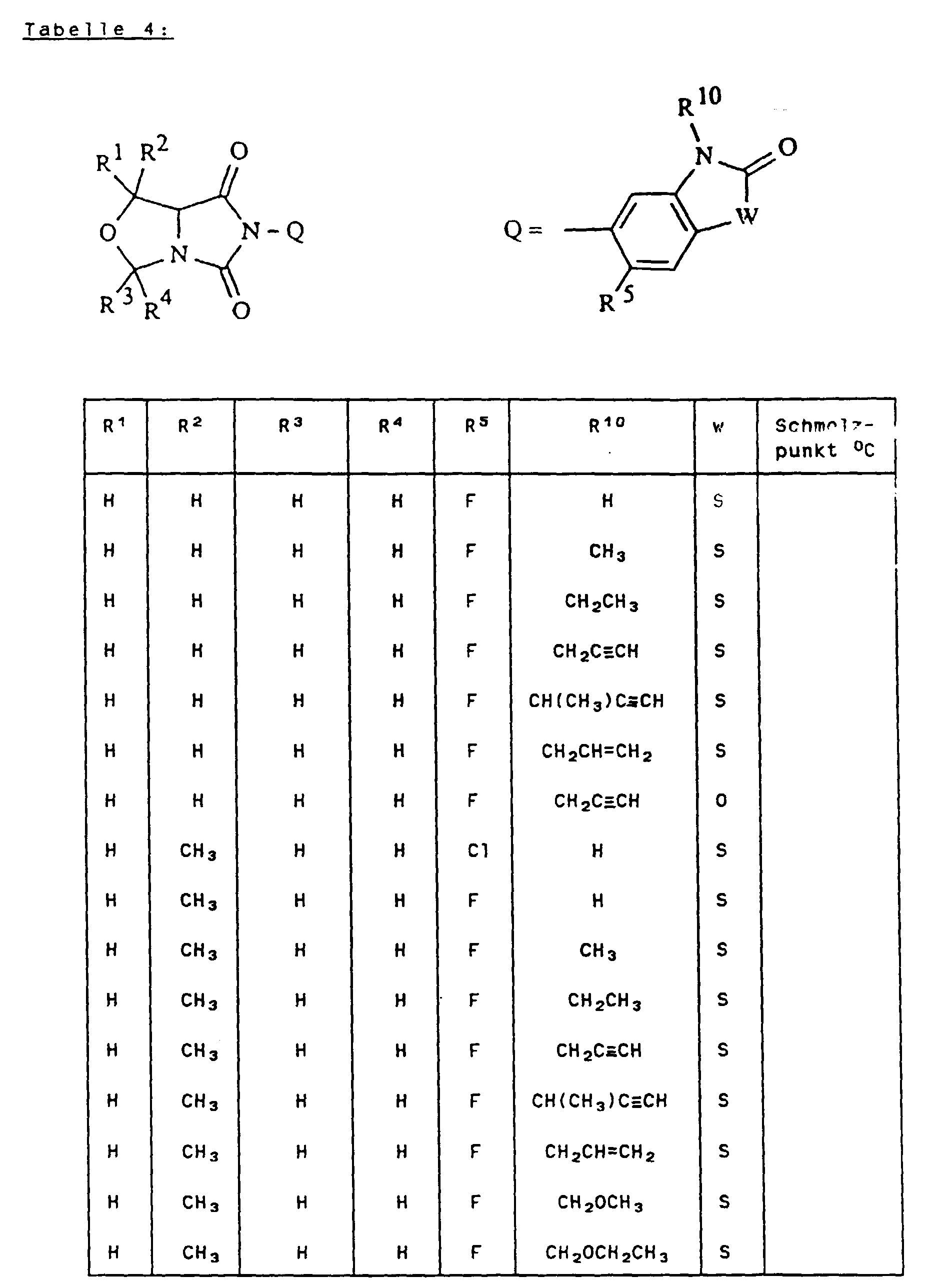

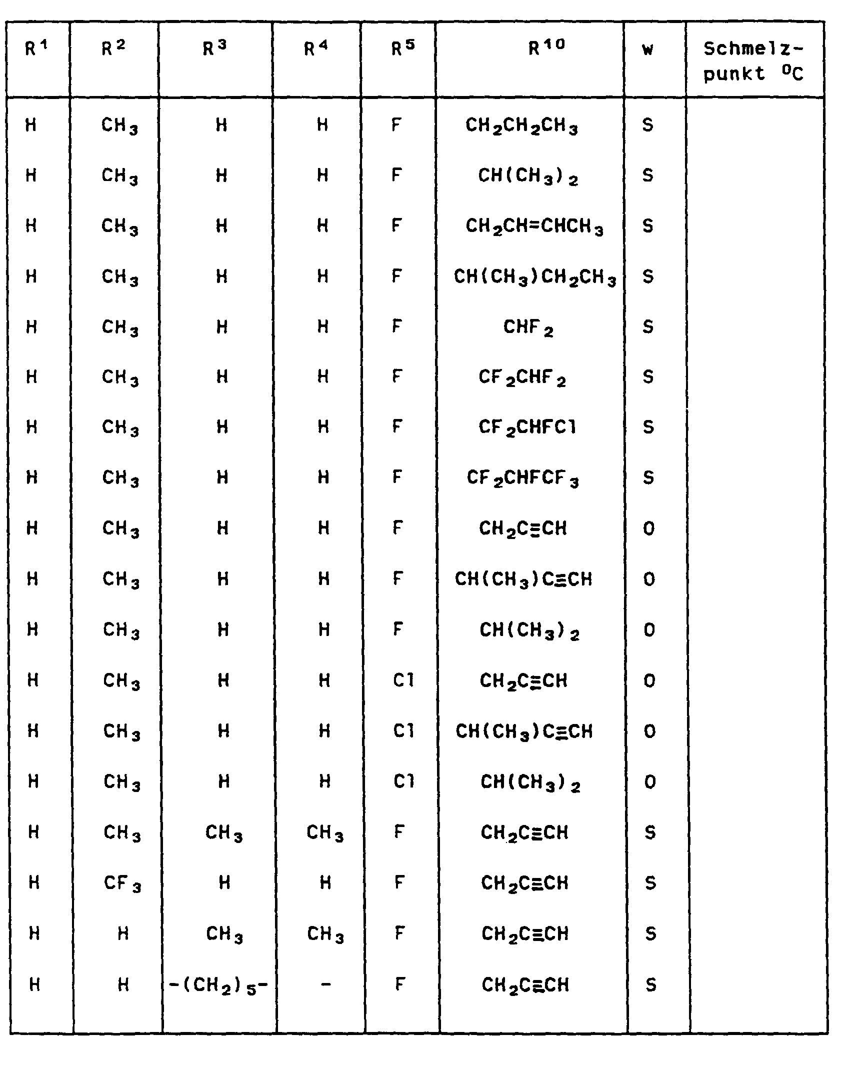

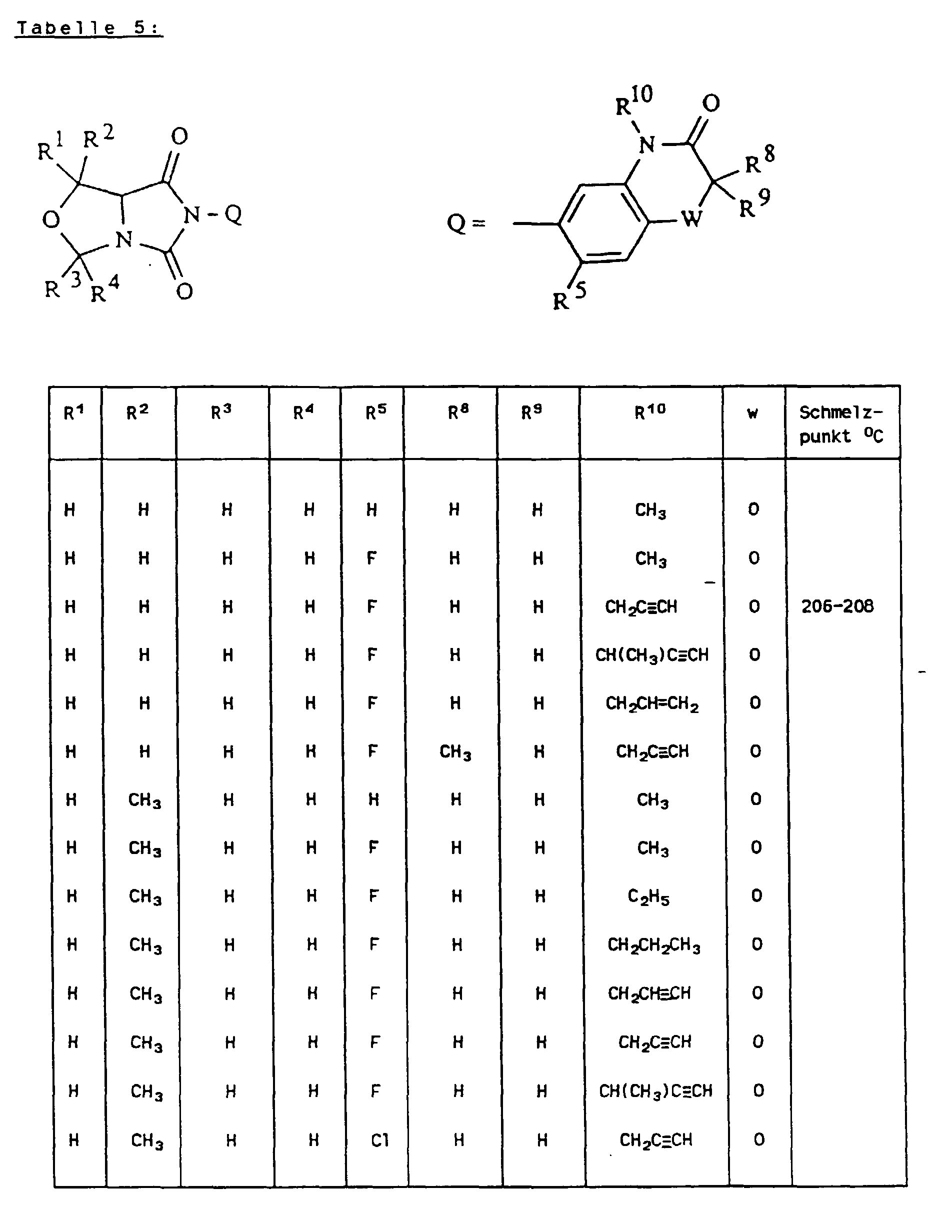

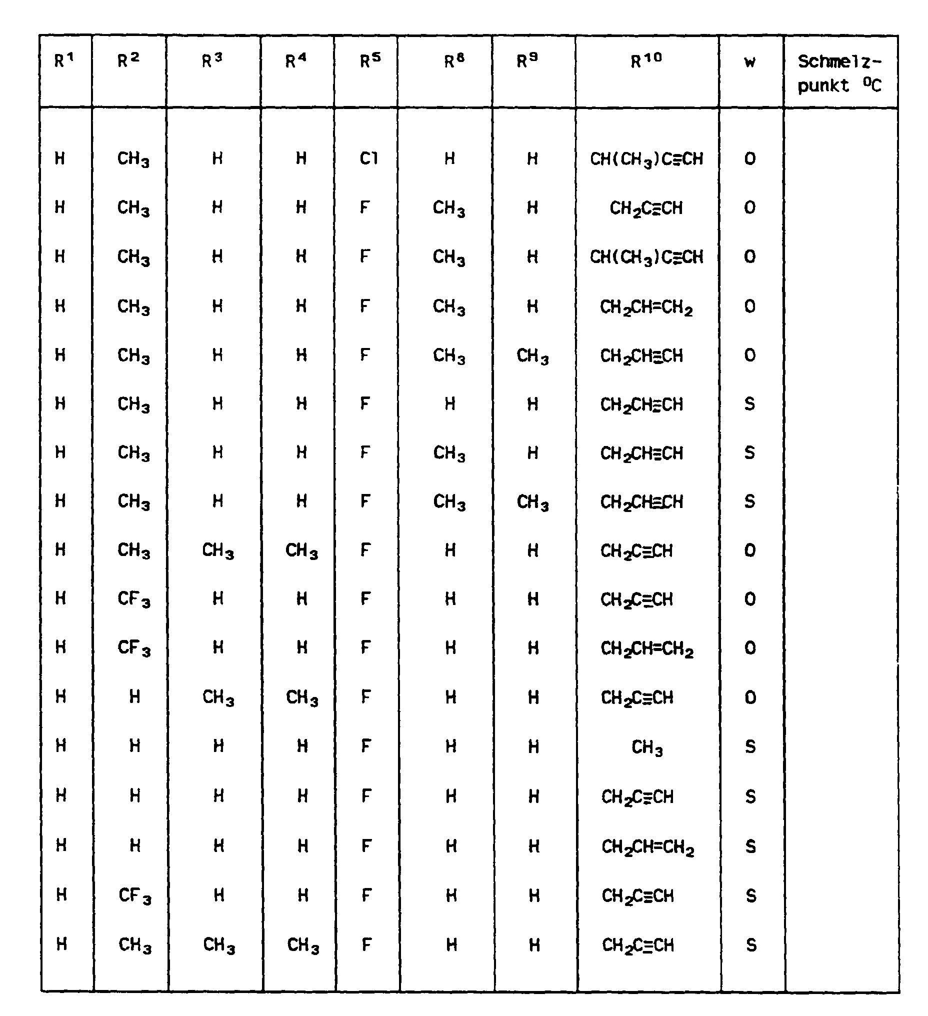

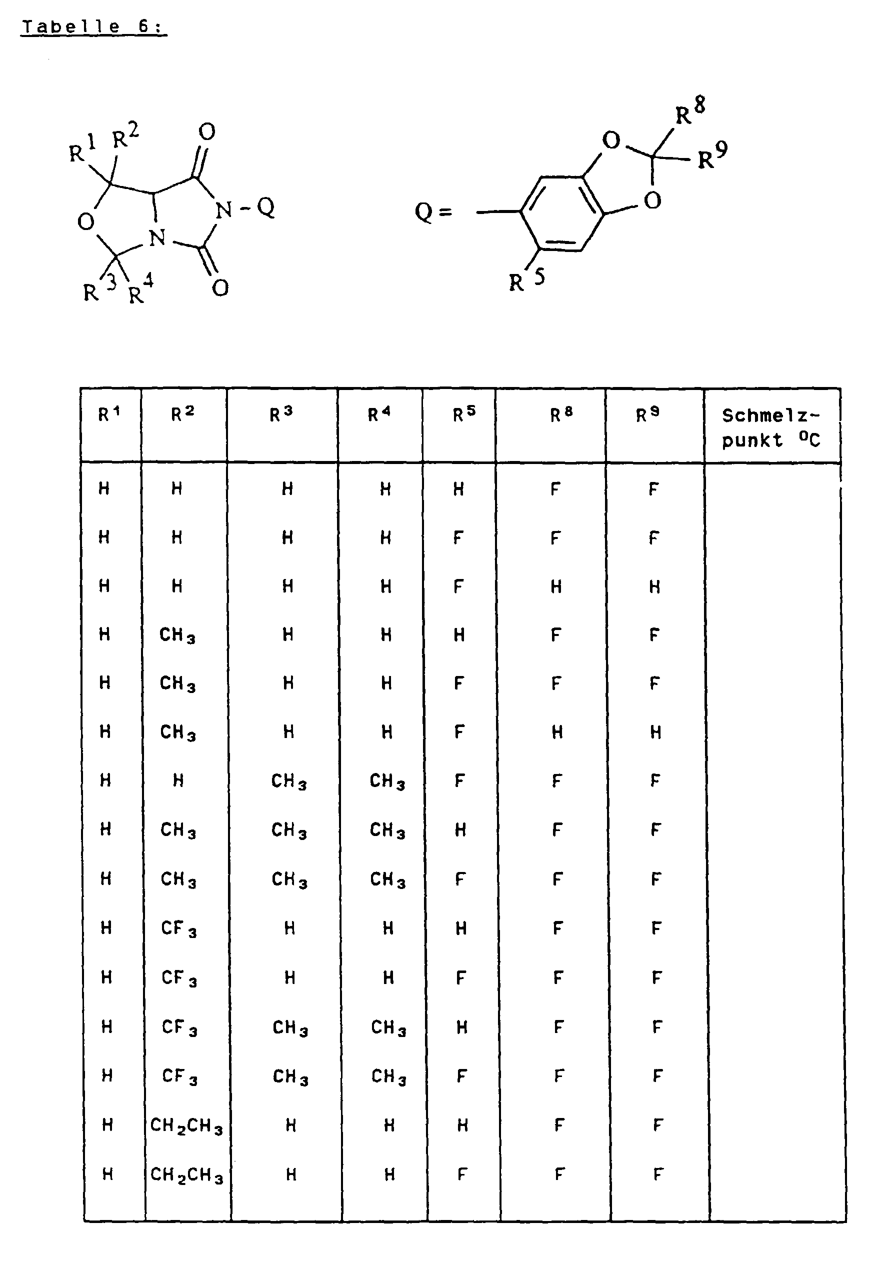

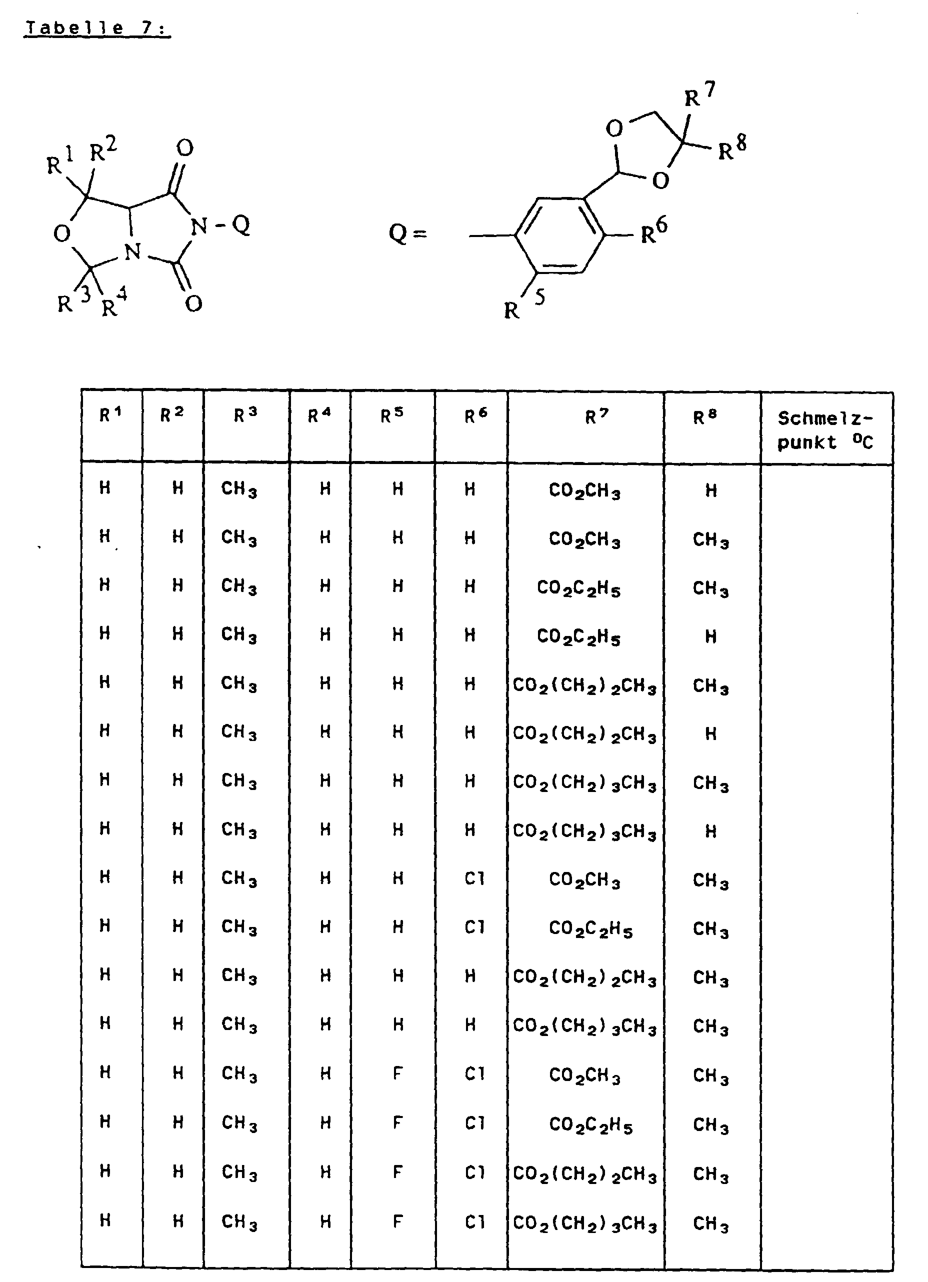

### Rezepturen

Zweckmäßige Rezepturen mit Verbindungen von Formel I können auf herkömmliche Weise, und zwar als Pulver, Granulat, Pellets, Lösungen, Suspensionen, Emulsionen, benetzbare Pulver, emulgierbare Konzentrate u. ä. hergestellt werden. Zahlreiche dieser Formen können direkt angewandt werden. Spritzbare Präparate können mit geeigneten Medien gestreckt werden und in Mengen zwischen einigen und einigen Hundert Litern pro Hektar verspritzt werden. Hochkonzentrierte Präparate werden vorwiegend als Zwischenprodukte für weitere Rezepturen verwendet. Die Rezepturen enthalten, grob gesagt, zwischen 0,1 und 99 Gew. % Wirkstoff(e) und zumindest einen Vertreter der Gruppe a) 0,1 bis 20 % oberflächenaktive Stoffe und b) etwa 1 bis 99,9 % fester oder flüssiger Verdünnungsmittel. Genauer gesagt, enthalten sie diese Bestandteile in annähernd den nachstehenden Mengen:

| | Wirkstoff | Gew%*) Verdünner | oberflächenaktive Stoffe |
|---|---|---|---|
| benetzte Pulver | 20 - 90 | 0 - 74 | 1 - 10 |
| Suspensionen in Öl, Emulsionen, Lösungen, (einschließlich emulgierbarer Konzentrate) | 3 - 50 | 40 - 95 | 0 - 15 |
| wäßrige Suspensionen | 10 - 50 | 40 - 84 | 1 - 20 |
| Stäube | 1 - 25 | 70 - 99 | 0 - 5 |
| Granulate und Pellets | 0,1 - 95 | 5 - 99,9 | 0 - 15 |
| hochkonzentrierte Präparate | 90 - 99 | 0 - 10 | 0 - 2 |

| | | | |
|---|---|---|---|
| * Wirkstoff plus zumindest ein oberflächenaktiver Stoff oder ein Verdünnungsmittel = 100 Gew% | | | |

Niedrigere oder höhere Wirkstoffgehalte können natürlich je nach vorgesehender Anwendung und den physikalischen Eigenschaften der Verbindung vorhanden sein. Höhere Mengenverhältnisse oberflächenaktive Komponente: Wirkstoff sind manchmal wünschenswert und werden durch Einarbeiten in die Rezeptur oder durch Mischen im Behälter erreicht.

Typische feste Verdünner werden beschrieben in Watkins, et al., "Handbook of Insecticide Dust Diluents and Carriers" (Handbuch der Verdünnungsmittel und Träger staubförmiger Insektizide), 2nd Ed., Dorland Books, Caldwell, New Jersey, doch können auch andere, entweder bergmännisch gewonnene oder industriell hergestellte Feststoffe verwendet werden. Für benetzbare Pulver werden die besser absorbierenden, für Stäube die dichteren Verdünner bevorzugt. Typische flüssige Verdünner und Lösemittel sind beschrieben in Marsden, "Solvents Guide" (Lösemittelführer), 2nd Ed., Interscience, New York, 1950. Für konzentrierte Suspensionen unter 0,1 % bevorzugt. Konzentrierte Lösungen sind vorzugsweise beständig gegen Phasentrennung bei 0°C. In "McCutchen's detergents and emulsifiers annual" (McCutcheon's Jahrbuch der Detergentien und Emulgatoren), MC Publishing Corp., Ridgewood, New Jersey, sowie in Sisely and Wood, "Encyclopedia of Surface Active Agents" (Enzyklopädie der oberflächenaktiven Stoffe), Chemical Publishing Co., Inc., New York, 1964, sind Listen von oberflächenaktiven Stoffen und dafür empfohlene Anwendungen enthalten. Alle Rezepturen können kleinere Mengen von Additiven zur Verringerung der Schaumbildung, des Zusammenbackens, der Korrosion, des Wachstums von Mikroorganismen u. s. w. enthalten.

Die Methoden zur Herstellung solcher Präparate sind gut bekannt. Lösungen werden hergestellt, indem man die Bestandteile einfach mischt. Feinpulverige feste Präparate werden durch Mischen und, gewöhnlich, Mahlen, z. B. in einer Hammermühle oder einer Strahlmühle, erhalten. Suspensionen werden durch Naßmahlen (siehe z. B. Littler, U.S. Patent 3,060,084) erhalten. Granulate und Pellets können hergestellt werden, indem man den Wirkstoff auf vorgeformte granulatförmige Träger aufsprüht oder indem man agglomeriert. Siehe hierzu J. E. Browning, "Agglomeration", Chemical Engineering, December 4, 1967, S. 147 ff, und "Perry's Chemlcal Engineer's Handbook" (Perry's Handbuch des chemischen Verfahrenstechnikers). 5th Ed., Mc Graw-Hill, New York, 1973, S. 8 - 57 ff.

Wegen weiterer Informationen bezüglich der Rezepturtechnik siehe z. B.:
H. M. Loux, U. S. Patent 3,235,361, 15. Februar, 1966, Spalte 6, Zelle 16 bis Spalte 7, Zelle 19 und Beispiele 10 bis 41;
R. W. Luckenbaugh, U. S. Patent 3,309,192, 14. März 1967, Spalte 5, Zeile 43 bis Spalte 7, Zeile 62 und Beispiele 8, 12, 15, 39, 41, 52, 53, 58, 132, 138 - 140, 162 - 164, 166, 167 und 169 - 182;
H. Gysin und E. Knusli, U. S. Patent 2,891,855, 23. Juni 1959, Spalte 3, Zeile 66 bis Spalte 5, Zelle 17 und Beispiele 1 - 4;
G. C. Klingman, "Weed Control as a Science" (Unkrautbekämpfung als Wissenschaft), John Wiley and Sons, Inc., New York, 1961, s. 81 -96 und
J. D. Fryer und S. A. Evans, "Weed Control Handbook" (Handbuch der Unkrautbekämpfung), 5th Ed., Blackwell Scientific Publications, Oxford, 1968, S. 101 - 103.

In den folgenden Beispielen handelt es sich um Gewichtsteile, sofern nichts anderes angegeben ist.

### Beispiel A

### Benetzbares Pulver

| | |
|---|---|
| 7-(4-Chlorphenyl)-5,5-dimethyl-6,8-dioxo-4-oxa-1,7-diazabicyclo[3.3.0]octan | 80 % |
| Natriumalkylnapthalinsulfonat | 2 % |
| Natriumlignosulfonat | 2 % |
| Synthetische amorphe Kieselsäure | 3 % |
| Kaolinit | 13 % |

Die Bestandteile werden gemischt und in einer Hammermühle gemahlen, bis alle Feststoffe im wesentlichen Korngrößen unter 50 µm haben, wonach erneut gemischt und verpackt wird.

### Beispiel B

### Benetzbares Pulver

| | |
|---|---|
| 7-(4-Chlorphenyl)-5,5-dimethyl-6,8-dioxo-4-oxa-1,7-diazabicyclo[3.3.0]octan | 50 % |
| Natriumalkylnaphthalinsulfonat | 2 % |
| Methylcellulose niedriger Viskosität | 2 % |
| Diatomeenerde | 46 % |

Die Bestandteile werden gemischt, in einer Hammermühle grob zerkleinert und dann in einer Strahlmühle gemahlen, so daß praktisch alle Teilchen unter 10 µm Durchmesser haben. Das Produkt wird dann vor dem Verpacken erneut gemischt.

### Beispiel C

### Granulat

| | |
|---|---|
| Benetzbares Pulver von Beispiel B | 5 % |
| Attapulgit-Granulat (USS 20 - 40 mesch; 0,84 - 0,42 mm) | 95 % |

Eine Aufschlämmung von benetzbarem Pulver mit 25 % Feststoffen wird in einen Doppelkonusmischer versprüht; die Granulen werden dann getrocknet und verpackt.

### Beispiel D

### Stranggepreßte Pellets

| | |
|---|---|
| 7-(4-Chlorphenyl)-5,5-dimethyl-6,8-dioxo-4-oxa-1,7-diazabicyclo[3.3.0]octan | 25 % |
| wasserfreies Natriumsulfat | 10 % |
| rohes Calciumlignosulfonat | 5 % |
| Natriumalkylnaphthalinsulfonat | 1 % |
| Calcium/Magnesium-Bentonit | 59 % |

Die Bestandteile werden gemischt, in einer Hammermühle gemahlen und dann mit etwa 12 % Wasser befeuchtet. Das Gemisch wird zu Zylindern mit etwa 3 mm Durchmesser stranggepreßt, die zu Pellets von etwa 3 mm Länge geschnitten werden. Diese können nach dem Trocknen direkt verwendet werden; man kann die getrockneten Pellets jedoch zerkleinern, so daß das USS No. 20-Sieb (Öffnungen 0,84 mm Durchmesser) hindurchgehen. Die auf dem Sieb USS No 40 (0,42 mm Öffnungsdurchmesser) zurückbleibenden Granulen können für die Verwendung verpackt werden, während die Feinanteile zurückgeführt werden.

### Beispiel E

### Granulat geringer Festigkeit

| | |
|---|---|
| 7-(4-Chlorphenyl)-5,5-dimethyl-6,8-dioxo-4-oxa-1,7-diazabicyclo[3.3.0]octan | 1 % |
| N,N-Dimethylformamid | 9 % |
| Attapulgit-Granulat (USS Siebe 20 bis 40) | 90 % |

Der Wirkstoff wird im Lösemittel gelöst und die Lösung auf entstaubte Granulen in einem Doppelkonusmischer gesprüht. Nach dem Versprühen der Lösung läßt man den Mischer noch eine kurze Zelt weiterlaufen, wonach die Granulen verpackt werden.

### Beispiel F

### Granulat

| | |
|---|---|
| 7-(4-Chlorphenyl)-5,5-dimethyl-6,8-dioxo-4-oxa-1,7-diazabicyclo[3.3.0]octan | 80 % |
| Netzmittel | 1 % |
| rohes Lignosulfonat (mit 5 bis 20 % der natürlichen Zucker) | 10 % |
| Attapulgit-Ton | 9 % |

Die Bestandteile werden gemischt und so weit gemahlen, daß sie durch ein 100 mesh-Sieb gehen. Dieses Material wird dann einem Fließbettgranulator zugeführt, wo der Luftstrom so eingestellt wird, daß das Material leicht aufgewirbelt wird, und wobei ein feiner Wasserstrahl auf das aufgewirbelte Material versprüht wird. Die Fluidisierung und das Sprühen werden so lange fortgesetzt, bis Granulen der gewünschten Größe erhalten werden. Das Sprühen wird dann eingestellt, das Fluidisieren hingegen, gegebenenfalls unter Wärmezufuhr, fortgesetzt, bis der Wassergehalt auf den gewünschten Wert, im allgemeinen unter 1 %, gesunken ist. Das Material wird dann abgezogen und der gewünschte Größenbereich, gewöhnlich 14 bis 100 mesh (1410 bis 149 µm) ausgesiebt, wonach für die Verwendung verpackt wird.

### Beispiel G

### Wäßrige Suspension

| | |
|---|---|
| 7-(4-Chlorphenyl)-5,5-dimethyl-6,8-dioxo-4-oxa-1,7-diazabicyclo[3.3.0]octan | 40 % |
| Verdickungsmittel auf Basis Polyacrylsäure | 0,3 % |
| Dodecylphenolpolyethylenglykolether | 0,5 % |
| Dinatriumphosphat | 1 % |
| Mononatriumphosphat | 0,5 % |
| Polyvinylalkohol | 1,0 % |
| Wasser | 56,7 % |

Die Bestandteile werden gemischt und gemeinsam in einer Sandmühle gemahlen, um Teilchen mit im wesentlichen unter 5 µm Größe zu erhalten.

### Beispiel H

### Starkes Konzentrat

| | |
|---|---|
| 7-(4-Chlorphenyl)-5,5-dimethyl-6,8-dioxo-4-oxa-1,7-diazabicyclo[3.3.0]octan | 99 % |
| Kieselsäure-Aerogel | 0,5 % |
| Synthetische amorphe Kieselsäure | 0,5 % |

Die Bestandteile werden gemischt und in einer Hammermühle gemahlen, um ein Material zu erhalten, das durch ein USS-Sieb Nr. 50 (0,3 mm Öffnung) hindurchgeht. Das Konzentrat kann, falls erforderlich, noch weitere Bestandteile enthalten.

### Beispiel I

### Benetzbares Pulver

| | |
|---|---|
| 7-(4-Chlorphenyl)-5,5-dimethyl-6,8-dioxo-4-oxa-1,7-diazabicyclo-[3.3.0]-octan | 90,0 % |
| Dioctylnatriumsulfosuccinat | 0,1 % |
| synthetische feine Kieselsäure | 9,9 % |

Die Bestandteile werden gemischt und in einer Hammermühle gemahlen, um Teilchen mit im wesentlichen unter 100 µm erhalten. Das Material wird durch ein USS Nr. 50 Sieb gesiebt und dann verpackt.

### Beispiel J

### Benetzbares Pulver

| | |
|---|---|
| 7-(4-Chlorphenyl)-5,5-dimethyl-6,8-dioxo-4-oxa-1,7-diazabicyclo-[3,3,0]-octan | 40 % |
| Natriumlignosulfonat | 20 % |
| Montmorillonit-Ton | 40 % |

Die Bestandteile werden gründlich gemischt, in einer Hammermühle grob gemahlen und dann in einer Luftstrahlmühle gemahlen, um Teilchen mit im wesentlichen unter 10 µm Größe zu erhalten. Das Materaial wird dann erneut gemischt und verpackt.

### Beispiel K

### Suspension in Öl

| | |
|---|---|
| 7-(4-Chlorphenyl)-5,5-dimethyl-6,8-dioxo-4-oxa-1,7-diazabicylo-[3.3.0]-octan | 35 % |
| Gemisch von Polyalkohol-Carboxylsäureestern und öllöslichen Petroleumsulfonaten | 6 % |
| Xylol | 59 % |

Die Bestandteile werden vermischt und in einer Sandmühle gemahlen, um Teilchen mit im wesentlichen unter 5 µm Größe zu erhalten. Das Produkt kann direkt, mit Öl gestreckt oder in Wasser emulgiert verwendet werden.

### Beispiel L

### Staub

| | |
|---|---|
| 7-(4-Chlorphenyl)-5,5-dimethyl-6,8-dioxo-4-oxa-1,7-diazabicyclo-[3.3.0]-octan | 10 % |
| Attapulgit | 10 % |
| Pyrophyllit | 80 % |

Der Wirkstoff wird mit Attapulgit gemischt und dann durch eine Hammermühle geschickt, um Teilchen mit im wesentlichen unter 200 µm zu erhalten. Das gemahlene Konzentrat wird dann mit dem pulverisierten Pyrophyllit bis zur Homogenität gemischt.

### Beispiel M

### Suspension in Öl

| | |
|---|---|
| 7-(4-Chlorphenyl)-5,5-dimethyl-6,8-dioxo-4-oxa-1,7-diatzabicyclo-[3.3.0]-octan | 25 % |
| Polyoxyethylensorbitolhexaoleat | 5 % |
| hochaliphatisches Kohlenwasserstofföl | 70 % |

Die Bestandteile werden gemeinam in einer Sandmühle gemahlen, bis die Größe der Feststoffteilchen unter etwa 5 µm liegt, resultierende dicke Suspension kann direkt verwendet werden, vorzugsweise verwendet man sie jedoch nach Strecken mit Ölen oder nach Emulgieren in Wasser.

### Biologische Beispiele

Versuchsergebnisse zeigen, daß die Verbindungen nach der vorliegenden Erfindung wirksame Herbizide sind. Sie eignen sich für die Breitband-Bekämpfung von Unkräutern vor und nach deren Keimen auf Flächen, wo die gesamte Vegetation unter Kontrolle gehalten werden soll, beisielsweise in der Umgebung von industriellen Lagerflächen, Parkplätzen, Drive-in-Kinos, um Reklamewände, an Landstraßen und an Eisenbahnbauten. Manche der Verbindungen eignen sich auch zur selektiven Unkrautbekämpfung in Kulturen wie Reis, Weizen, Gerste, Mais, Sojabohnen, Zuckerrüben und Baumwolle.

Die aufzubringende Menge der Verbindungen nach der vorliegenden Erfindung ist von zahlreichen Faktoren abhängig, darunter der Verwendung als selektive oder universelle Herbizide, der jeweiligen Feldfrucht, der Art der zu bekämpfenden Unkräuter, dem Wetter und Klima, der ausgewählten Rezeptur, der Anwendungsweise, der Menge der Blattpflanzen u.a. Im allgemeinen sollten die Verbindungen in Mengen zwischen 0,001 und 20 kg/ha aufgebracht werden, wobei die niedrigeren Mengen für leichtere Böden und/oder Böden mit niedrigem Gehalt an organischen Stoffen oder für Fälle infrage kommen, wo nur kurze Verweilzeit erforderlich ist, beispielswiese im Falle von Herbiziden für Brachland.
Die Verbindungen nach der vorliegenden Erfindung können in Kombinationen mit jedem anderen handelsüblichen Herbizid verwendet werden.

Die herbiziden Eigenschaften der Verbindungen nach der vorliegenden Erfindung wurden bei einer Reihe von Treibhaus-Versuchen gefunden. Die Prüfverfahren und -ergebnisse werden im folgenden angegeben.

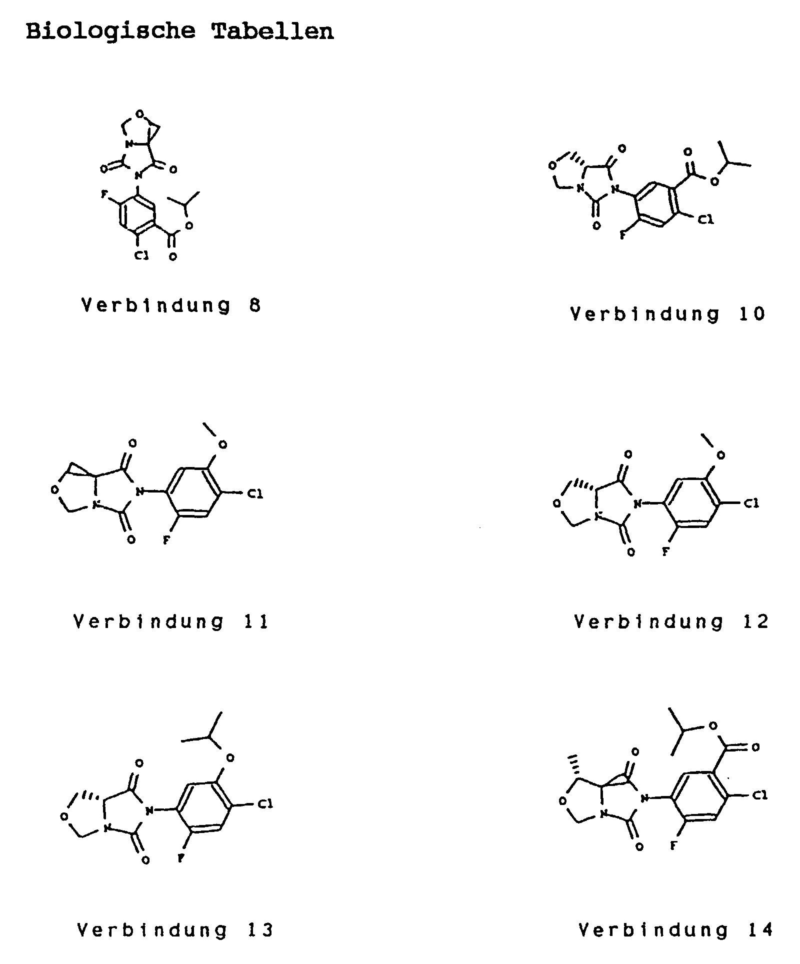

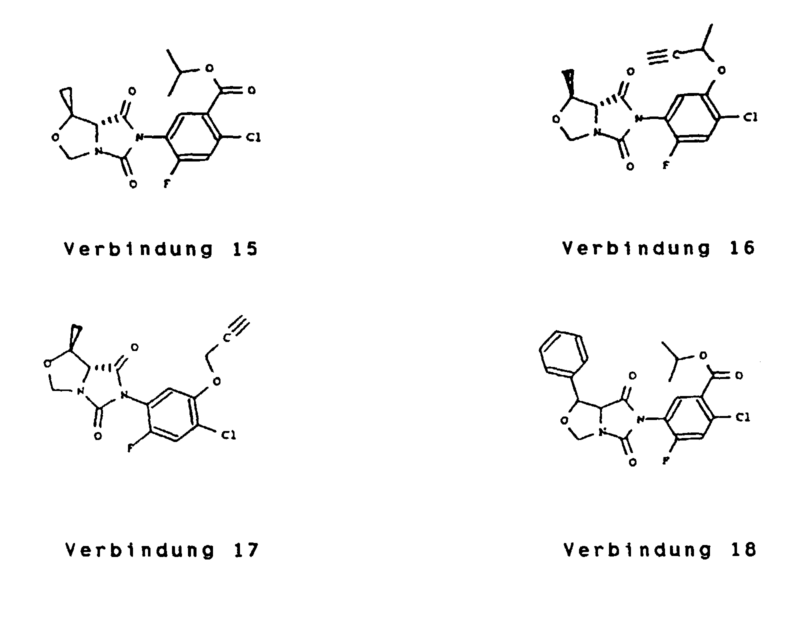

### Prüfverfahren

Samen von digitaria spp, echinochloa crus-galli, setaria feberil, avena fatua, bromus secalinus, abutilon theophrasti, ipomoea spp., xanthlum pensylvanicum und Sorghum-Knollen wurden gesetzt und vor dem Keimen mit den in einem nicht-phytotoxischen Lösemittel gelösten Versuchschemikalien behandelt.

Außerdem wurden diese Unkrautarten mit einem für den Boden und das Blattwerk bestimmten Präparat behandelt. Zum Zeitpunkt der Behandlung hatten die Pflanzen 2 bis 18 cm Höhe. Die behandelten Pflanzen und die Kontrollpflanzen wurden 16 d lang im Treibhaus gehalten, wonach alle Exemplare mit den Kontrollpflanze wurden 16 d lang im Treibhaus gehalten, wonach alle Exemplare mit den Kontrollpflanzen verglichen und die Wirkung der Behandlung visuell beurteilt wurde. Die in Tab. A zusammengestellten Bewertungen auf einer numerischen Skala von 0 = ohne Schaden bis 10 = vollständige Vernichtung.

Die beigegebenen beschreibenden Symbole haben die nachstehenden Bedeutungen:
C = Chlorose/Nekrose
B = Verbrennung
H = formbildende Wirkung
E = Keimhemmung
G = Wachstumsförderung

**Tabelle A**

| Anwendung nach dem Keimen (Dosis 2 kg Wirkstoff/ha) | | | | | | | | | | |
|---|---|---|---|---|---|---|---|---|---|---|
| | Verb. 8 | Verb. 10 | Verb. 11 | Verb. 12 | Verb. 13 | Verb. 14 | Verb. 15 | Verb. 16 | Verb. 17 | Verb. 18 |
| Echinocloa c.-g. | 7B | 10B | 2B | 9B | 10B | 9B | 10B | 9 | 10 | 3 |
| Bromus secalinus | 2B | 10B | 1B | 5B | 9B | 9B | 10B | - | - | - |
| Xanthium pens. | 1B | - | - | - | - | - | - | - | - | - |
| Ipomoea spp. | 8B | 10B | 8B | 10B | 10B | 10B | 10B | 10 | 10 | |
| Sorghum | 3B | 10B | 3B | 8B | 9B | 4B | 10B | 10 | 10 | 8 |
| Setaria feberii | 7B | 10B | 3B | 9B | 10B | 10B | 10B | 10 | 10 | 6 |
| Digitaria spp. | 7B | 10B | 3B | 9B, 6H | 10B | 9B | 10B | 10 | 10 | 7 |
| Abutilon th. | 8B | 10B | 4B | 10B | 10B | 10B | 10B | 10 | 10 | 6 |
| Avena fatua | 3B | 10B | 2B | 6B | 9B | 9B | 10B | 10 | 10 | 7 |

**Tabelle B**

| Anwendung vor dem Keimen (Dosis 2 kg Wirkstoff/ha) | | | | | | | | | | |
|---|---|---|---|---|---|---|---|---|---|---|
| | Verb. 8 | Verb. 10 | Verb. 11 | Verb. 12 | Verb. 13 | Verb. 14 | Verb. 15 | Verb. 16 | Verb. 17 | Verb. 18 |
| Echinocloa c.-g. | 6H | 9C | 4C | 9C | 10C | 9C | 10C | - | - | - |
| Bromus secalinus | 2G | 9C | 3C | 8C | 9C | 9C | 10C | - | - | - |
| Xanthium pens. | 2G | - | - | - | - | - | - | - | - | - |
| Ipomoea spp. | 0 | 10C | 4C | 10C | 9C | 10C | 10C | 10 | 10 | 9 |
| Sorghum | 0 | 10C | 3C | 10C | 9C | 8C | 10C | 10 | 10 | 7 |
| Setaria feberii | 9H | 10C | 7C, 3H | 10C | 10C | 10C | 10C | 10 | 10 | 9 |
| Digitaria spp. | 9H | 10C | 6C | 9C | 9C | 10C | 10C | 10 | 10 | 7 |
| Abutilon th. | 10C | 10C | 10C | 10C | 10C | 10C | 10C | 10 | 10 | 7 |
| Avena fatua | 0 | 9C | 1C | 9C | 9C | 8C | 10C | 10 | 10 | 3 |

**Tabelle C**

| Anwendung nach dem Keimen (Dosis 0,2 kg/ha) | | | | | | |
|---|---|---|---|---|---|---|
| Objekt | Verb. 10 | Verb. 12 | Verb. 13 | Verb. 15 | Verb. 16 | Verb. 17 |
| Mais | 9B | 2B | 2B | 6B | 5B | 5B |
| Weizen | 4B | 2B | 3B | 7B | 5B | 7B |
| Echinocloa c.-g. | 9B | 3B | 9B | 10B | 8B | 9B |
| Xanthium pens. | 7B | - | - | - | 6B | 4B |
| Ipomoea spp. | 10B | 8B | 9B | 19B | 8B | 8B |
| Sorghum | 4B | 2B | 3B | 7B | 7B | 7B |
| Setaria feberii | 7B | 4B | 6B | 10B | 8B | 9B |
| Digitaria spp. | 3B | 2B | 3B | 9B | 8B | 7B |
| Abutilon th. | 10B | 9B | 9B | 10B | 9B | 10B |
| Avena fatua | 3B | 2B | 3B | 7B | 5B | 5B |

**Tabelle D**

| Anwendung vor dem Keimen (Dosis 0,2 kg/ha) | | | | | | |
|---|---|---|---|---|---|---|
| Objekt | Verb. 10 | Verb. 12 | Verb. 13 | Verb. 15 | Verb. 16 | Verb. 17 |
| Mais | 1C | 0 | 0 | 2C | 6H | 6H |
| Weizen | 0 | 0 | 2G | 1G | 2C | 2C |
| Echinocloa c.-g. | 1C | 1C | 6C | 10C | 9H | 9H |
| Xanthium pens. | 7B | - | - | - | 2H | 0 |
| Ipomoea spp. | 3C | 1C | 7C | 10C | 5H | 10E |
| Sorghum | 1C | 0 | 2C | 4C | 8H | 5G |
| Setaria feberii | 9C | 6C | 7C | 10C | 10H | 10H |
| Digitaria spp. | 9G | 3C | 4C | 10C | 10H | 10H |
| Abutilon th. | 10C | 10C | 10C | 10C | 10C | 10C |
| Avena fatua | 2C | 1C | 0 | 4C | 7H | 7H |

## Patentansprüche

1. Anellierte (Oxa)hydantoine der Formel I, 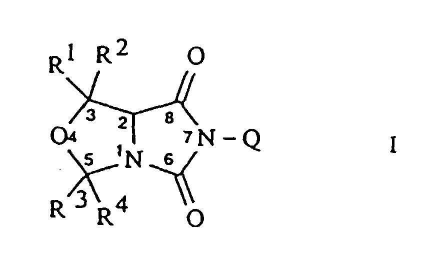 worin R¹ und R², unabhängig voneinander, für Wasserstoff oder ein Rest aus der Reihe (C₁ - C₄)Alkyl, (C₁ - C₂)Haloalkyl oder Phenyl gegebenenfalls fluorsubstituiert, stehen,
R³ und R⁴, unabhängig voneinander, für Wasserstoff, (C₁ - C₄)Alkyl, Phenyl, beide gegebenenfalls fluor- und/oder chlor-, brom- oder methylsubstituiert, oder (C₁ - C₄)Alkoxy stehen, oder zusammen einen carbocyclischen Ring bilden, der gegebenenfalls durch (C₁ - C₄)Alkyl substituiert sein kann;
Q für einen der Reste Q₁ - Q₇ steht 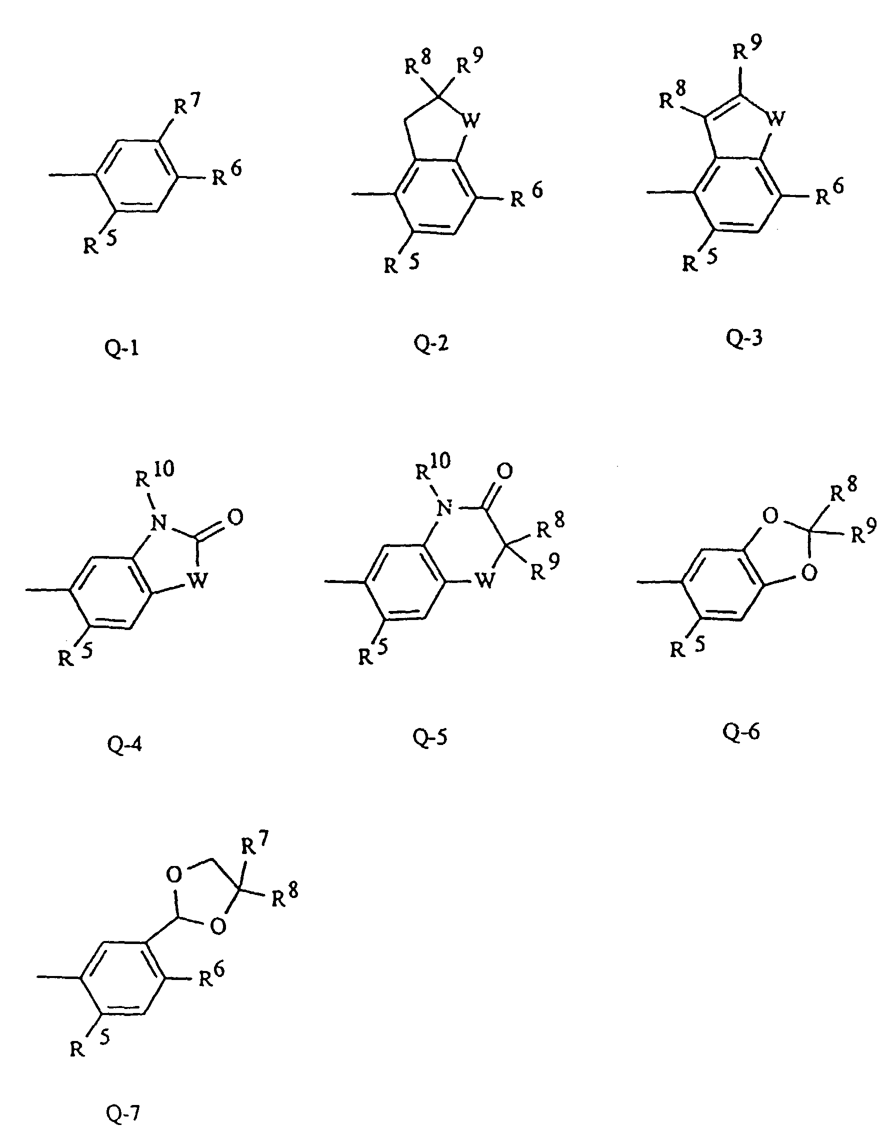 worin
W für O oder S steht,
R⁵ für Wasserstoff oder Halogen steht,
R⁶ für (C₁ - C₂)Alkyl, (C₁ - C₂)Haloalkyl, OCH₃, SCH₃, OCHF₂, Halogen, CN oder NO₂ steht,
R⁷ für (C₁ - C₈)Alkyl, (C₁ - C₈)Haloalkyl, Halogen, OR¹¹, S(O)ₙR¹¹, COR¹¹, CO₂R¹¹, C(O)SR¹¹, C(O)NR¹²R¹³, CHO, CH=CHCO₂R¹¹, CO₂N=CR¹⁴R¹⁵, NO₂, CN, NHSO₂R¹⁶ oder NHSO₂NHR¹⁶ steht,
R⁸ für Wasserstoff, (C₁ - C₃)Alkyl, (C₁ - C₃)Haloalkyl oder Halogen steht,
R⁹ für Wasserstoff, (C₁ - C₃)Alkyl, (C₁ - C₃)Haloalkyl oder Halogen steht; oder wenn Q = Q-2 oder Q-6 ist, können R⁸ und R⁹ zusammen mit dem Kohlenstoffatom, an das sie gebunden sind, C=O sein;
R¹⁰ für (C₁ - C₆)Alkyl, (C₁ - C₆)Haloalkyl, (C₂ - C₆)Alkoxyalkyl, (C₃ - C₆)Alkenyl oder (C₃ - C₆)Alkinyl steht,
R¹¹ für (C₁ - C₈)Alkyl, (C₃ - C₈)Cycloalkyl, (C₃ - C₈)Alkenyl, (C₃ - C₈)Alkinyl, (C₁ - C₈)Haloalkyl, (C₂ - C₈)Alkoxyalkyl, (C₂ - C₈)Alkylthioalkyl, (C₂ - C₈)Alkylsulfinylalkyl, (C₂ - C₈) Alkylsulfonylalkyl, (C₃ - C₈)Alkoxyalkoxyalkyl, (C₄ - C₈)Cycloalkylalkyl, (C₂ - C₄)Carboxyalkyl, (C₃ - C₈)Alkoxycarbonylalkyl, (C₆ - C₈)Alkenyloxycarbonylalkyl, (C₆ - C₈)Alkinyloxycarbonylalkyl, (C₄ - C₈)Alkenoxyalkyl, (C₆ - C₈)Cycloalkoxyalkyl, (C₄ - C₈)Alkinyloxyalkyl, (C₃ - C₈)Haloalkoxyalkyl, (C₄ - C₈)Haloalkenyloxyalkyl, (C₄ - C₈)Haloalkinyloxyalkyl, (C₆ - C₈)Cycloalkylthioalkyl, (C₄ - C₈)Alkenylthioalkyl, (C₄ - C₈)Alkinylthioalkyl, (C₁ - C₄)Alkyl substituiert mit Phenoxy oder Benzyloxy, die beide gegebenenfalls Halogen, (C₁ - C₃)Alkyl oder (C₁ - C₃)Haloalkylsubstituiert sein können; (C₄ - C₈)Trialkylsilylalkyl, (C₃ -C₈)Cyanoalkyl, (C₃ - C₈)Halocycloalkyl, (C₃ - C₈)Haloalkenyl, (C₅ - C₈)Alkoxyalkenyl, (C₅ - C₈)Haloalkoxyalkenyl, (C₅ - C₈)Alkylthioalkenyl, (C₃ - C₈)Haloalkinyl, (C₅ - C₈)Alkoxyalkinyl, (C₅ - C₈)Haloalkoxyalkinyl, (C₅ - C₈)Alkylthioalkinyl, (C₂ - C₈)Alkylcarbonyl, Benzyl gegebenenfalls substituiert mit Halogen, (C₁ - C₃)Alkyl oder (C₁ - C₃)Haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, Phenyl oder Pyridyl, beide gegebenenfalls substituiert mit Halogen, (C₁ - C₃)Alkyl, (C₁ - C₃)Haloalkyl oder (C₁ - C₄)Alkoxy, steht,
R¹² und R¹⁴, unabhängig voneinander, für Wasserstoff oder (C₁ - C₄)Alkyl stehen,
R¹³ und R¹⁵, unabhängig voneinander, für (C₁ - C₄)Alkyl, Phenyl, gegebenenfalls substituiert mit Halogen, (C₁ - C₃)Alkyl, (C₁ - C₃)Haloalkyl oder (C₁ - C₄)Alkoxy, stehen, oder
R¹² und R¹³ als -(CH₂)₅-, -(CH₂)₄- oder -CH₂CH₂OCH₂CH₂- zu Ringen kombiniert sein können, wobei ein oder mehrere H-Atome in jedem Ring gegebenenfalls durch (C₁ - C₃)Alkyl, Phenyl- oder Benzyl substituiert sein können;
R¹⁴ und R¹⁵ zusammen mit dem Kohlenstoffatom, an den sie gebunden sind, eine (C₃ - C₈)Cycloalkylgruppe bilden können,
R¹⁶ für (C₁ - C₄)Alkyl oder (C₁ - C₄)Haloalkyl steht,
R¹⁷ für Wasserstoff oder (C₁ - C₃)Alkyl steht,
R¹⁸ für (C₁ - C₆)Alkyl, (C₃ - C₆)Alkenyl oder (C₃ - C₆)Alkinyl steht, und
n für 0, 1, 2 steht.

2. Anellierte (Oxa)-hydantoine der Formel I nach Anspruch I,
**dadurch gekennzeichnet,**
daß mindestens einer der Reste
R¹ und R², unabhängig voneinander, für Wasserstoff oder eine Gruppe der Reihe (C₁ - C₄)Alkyl, (C₁ - C₂)Haloalkyl oder Phenyl, das gegebenenfalls fluorsubstituiert ist, stehen,
R³ und R⁴, unabhängig voneinander, für Wasserstoff, (C₁ - C₃) Alkyl, Phenyl, gegebenenfalls fluorsubstituiert und/oder chlor-, brom, methylsubstituiert, (C₁ - C₂)Alkoxy stehen oder zusammen einen carbocyclischen Ring bilden, der gegebenenfalls (C₁ - C₂)alkylsubstituiert sein kann,
W für O oder S steht,
n für 0, 1, 2 steht,
R⁵ für Wasserstoff oder Halogen steht,
R⁶ für Halogen oder CN steht,
R⁷ für (C₁ - C₄)Alkyl, (C₁ - C₄)Haloalkyl, Halogen, OR¹¹, S(O)ₙR¹¹, COR¹¹, CO₂R¹¹, C(O)SR¹¹, C(O)NR¹²R¹³, CH=CHCO₂R¹¹, CO₂N=CR¹⁴R¹⁵, NHSO₂R¹⁶ oder NHSO₂NHR¹⁶ steht,
R⁸ für Wasserstoff, (C₁ - C₃)Alkyl oder (C₁ - C₃)Haloalkyl steht,
R⁹ für Wasserstoff, (C₁ - C₃)Alkyl oder (C₁ - C₃)Haloalkyl steht; oder wenn Q = Q-2 oder Q-6 ist, können R⁸ and R⁹ zusammen mit dem Kohlenwasserstoffatom, an das sie gebunden sind, C=O sein;
R¹⁰ für (C₁ - C₄)Alkyl, (C₁ - C₄)Haloalkyl, (C₂ - C₄)Alkoxyalkyl, (C₃ - C₆)Alkenyl oder (C₃ - C₆)Alkinyl steht,
R¹¹ für (C₁ - C₄)Alkyl, (C₃ - C₆)Cycloalkyl, (C₃ - C₆)Alkenyl, (C₃ - C₆)Alkinyl, (C₁ - C₄)Haloalkyl, (C₂ - C₄)Alkoxyalkyl, (C₂ - C₄)Alkylthioalkyl, (C₂ - C₄)Alkylsulfinylalkyl, (C₂ - C₄)Alkylsulfonylalkyl, (C₃ - C₆)Alkoxyalkoxyalkyl, (C₄ - C₈)Cycloalkylalkyl, (C₂ - C₄)Carboxyalkyl, (C₃ - C₆) Alkoxycarbonylalkyl, (C₆ - C₈)Alkenyloxycarbonylalkyl, (C₆ - C₈)Alkinyloxycarbonylalkyl, (C₄ - C₆)Alkenoxyalkyl, (C₆ - C₈)Cycloalkoxyalkyl, (C₄ - C₆)Alkinyloxyalkyl, (C₃ - C₆)Haloalkoxyalkyl, (C₄ - C₈)Haloalkenyloxyalkyl, (C₄ - C₆)Haloalkinyloxyalkyl, (C₆ - C₈)Cycloalkylthioalkyl, (C₄ - C₆)Alkenylthioalkyl, (C₄ - C₆)Alkinylthioalkyl, (C₁ - C₂)Alkyl substituiert mit Phenoxy oder Benzyloxy, die beide gegebenenfalls mit Halogen, (C₁ - C₃)Alkyl oder (C₁ - C₃)Haloalkyl substituiert sein können; (C₄ - C₈)Trialkylsilylalkyl, (C₃ - C₄)Cyanoalkyl, (C₃ - C₆)Halocycloalkyl, (C₃ - C₆)Haloalkenyl, (C₅ - C₆)Haloalkoxyalkenyl, (C₅ - C₆)Alkylthioalkenyl, (C₃ - C₆)Haloalkinyl, (C₅ - C₆)Alkoxyalkinyl, (C₅ - C₆)Haloalkoxyalkinyl, (C₅ - C₆)Alkylthioalkinyl, (C₂ - C₄)Alkylcarbonyl, Benzyl gegebenenfalls substituiert mit Halogen, (C₁ - C₂)Alkyl oder (C₁ - C₂)Haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, Phenyl oder Pyridyl, beide gegebenenfalls substituiert mit Halogen, (C₁ - C₃)Alkyl, (C₁ - C₃)Haloalkyl oder (C₁ - C₄)Alkoxy, steht,
R¹² und R¹⁴, unabhängig voneinander, für Wasserstoff oder (C₁ - C₂)Alkyl stehen,
R¹³ und R¹⁵, unabhängig voneinander, für (C₁ - C₂)Alkyl, Phenyl gegebenenfalls substituiert mit Halogen, (C₁ - C₂)Alkyl, (C₁ - C₂)Haloalkyl oder (C₁ - C₂)Alkoxy, stehen, oder
R¹² und R¹³ als -(CH₂)₅-, -(CH₂)₄- oder -CH₂CH₂OCH₂CH₂- zu Ringen kombiniert sein können, wobei ein oder mehrere H-Atome in jedem Ring gegebenenfalls durch (C₁ - C₂)Alkyl-, Phenyl- oder Benzyl subsituiert sein können,
R¹⁴ und R¹⁵ zusammen mit dem Kohlenstoff, an den sie gebunden sind, eine (C₃ - C₆)Cycloalkylgruppe bilden können,
R¹⁶ für (C₁ - C₄)Alkyl oder (C₁ - C₄)Haloalkyl steht,
R¹⁷ für Wasserstoff oder (C₁ - C₃)Alkyl steht,
R¹⁸ für (C₁ - C₄)Alkyl, (C₃ - C₄)Alkenyl oder (C₃ - C₄)Alkinyl steht.

3. Anellierte (Oxa)-hydantoine der Formel I nach Anspruch 1,
**dadurch gekennzeichnet,**
daß mindestens einer der Reste
R¹ und R², unabhängig voneinander, für Wasserstoff oder einen Rest der Reihe (C₁ - C₃)Alkyl, (C₁ - C₂)Haloalkyl oder Phenyl, das gegebenenfalls floursubstituiert ist, stehen,
R³ und R⁴, unabhängig voneinander, für Wasserstoff, (C₁ - C₃) Alkyl stehen, oder auch zusammen einen 5 - 6 gliedrigen carbocyclischen Ring bilden, der gegebenenfalls (C₁ - C₄)alkylsubstituiert sein kann,
W für O oder S steht,
R⁵ für Wasserstoff, Fluor oder Chlor steht,
R⁶ für Chlor, Brom oder Cyan steht,
R⁷ für OR¹¹ oder CO₂R¹¹ steht,
R⁸ und R⁹, unabhängig voneinander, für Wasserstoff, (C₁ - C₂)Alkyl oder (C₁ - C₂)Haloalkyl stehen,
R¹⁰ für (C₁ - C₂)Alkyl, (C₁ - C₂)Haloalkyl, (C₃ - C₄)Alkenyl oder (C₃ - C₄)Alkinyl stehen,
R¹¹ für (C₁ - C₄)Alkyl, (C₃ - C₆)Cycloalkyl, (C₃ - C₆)Alkenyl, (C₃ - C₆)Alkinyl, (C₁ - C₄)Haloalkyl, (C₂ - C₄)Alkoxyalkyl, (C₂ - C₄)Alkylthioalkyl, (C₂ - C₄)Alkylsulfinylalkyl, (C₂ - C₄) Alkylsulfonylalkyl (C₃ - C₆)Alkoxyalkoxyalkyl, (C₄ - C₈) Cycloalkylalkyl, (C₂ - C₄)Carboxyalkyl, (C₃ - C₆)Alkoxycarbonylalkyl, (C₆ - C₈)Alkenyloxycarbonylalkyl, (C₆ - C₈) Alkinyloxycarbonylalkyl, (C₆ - C₈)Cycloalkoxyalkyl, (C₄ - C₆)Alkenyloxyalkyl, (C₄ - C₆)Alkinyloxyalkyl, (C₃ - C₆)Haloalkoxyalkyl, (C₄ - C₈)Haloalkenyloxyalkyl, (C₄ - C₆)Haloalkinyloxyalkyl, (C₆ - C₈)Cycloalkylthioalkyl, (C₄ - C₆)Alkenylthioalkyl, (C₄ - C₆)Alkinylthloalkyl, (C₁ - C₂)Alkyl substituiert mit Phenoxy oder Benzyloxy, die beide gegebenenfalls substituiert sind mit Halogen, (C₁ - C₃)Alkyl oder (C₁ - C₃)Haloalkyl; (C₄ - C₈)Trialkylsilylalkyl, (C₃ - C₄)Cyanoalkyl, (C₃ - C₆)Halocycloalkyl, (C₃ - C₆)Haloalkenyl, (C₅ - C₆)Alkoxyalkenyl, (C₅ - C₆)Haloalkoxyalkenyl, (C₅ - C₆)Alkylthioalkenyl, (C₃ - C₆)Haloalkinyl , (C₅ - C₆)Alkoxyalkinyl, (C₅ - C₆)Haloalkoxyalkinyl, (C₅ - C₆)Alkylthioalkinyl, (C₂ - C₄)Alkylcarbonyl, Benzyl gegebenenfalls substituiert mit Halogen, (C₁ - C₂)Alkyl oder (C₁ - C₂)Haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NRⁱ²Rⁱ³, CHR¹⁷C(O)NH₂, Phenyl oder Pyridyl, beide gegebenenfalls substituiert mit Fluor, Chlor, Brom, (C₁ - C₂)Haloalkyl oder (C₁ - C₂)Alkoxy, steht,
R¹² für Wasserstoff oder (C₁ - C₂)Alkyl steht,
R¹³ für (C₁ - C₂)Alkyl, Phenyl gegebenenfalls substituiert mit Fluor, Chlor, Brom, (C₁ - C₂)Alkyl, (C₁ - C₂)Haloalkyl oder (C₁ - C₂)Alkoxy, steht, oder
R¹² und R¹³ können als -(CH₂)₅-, -(CH₂)₄- oder -CH₂CH₂OCH₂OH₂- zu Ringen kombiniert sein, wobei ein oder mehrere H-Atome in jedem Ring gegebenenfalls durch (C₁ - C₂)Alkyl substituiert sein können,
R¹⁷ für Wasserstoff oder (C₁ - C₂)Alkyl steht,
R¹⁸ für (C₁ - C₂)Alkyl, (C₃ - C₄)Alkenyl oder (C₃ - C₄)Alkinyl steht.

4. Anellierte (Oxa)-hydantoine der Formel I nach Anspruch 1,
**dadurch gekennzeichnet,**
daß mindestens einer der Reste
R¹ und R², unabhängig voneinander, für Wasserstoff, (C₁ - C₃)Alkyl, (C₁ - C₂)Haloalkyl oder Phenyl stehen,
R³ und R⁴, unabhängig voneinander, für Wasserstoff oder (C₁ - C₃)Alkyl stehen oder auch zusammen einen 5 - 6 gliedrigen carbocyclischen Ring bilden können,
R⁵ für Fluor oder Chlor steht,
R⁶ für Chlor steht,
R⁷ für OR¹¹ oder CO₂R¹¹ steht,
R¹¹ für (C₁ - C₄)Alkyl, (C₃ - C₆)Cycloalkyl, (C₃ - C₆)Alkenyl, (C₃ - C₄)Alkinyl, (C₁ - C₃)Haloalkyl, (C₂ - C₄)Alkoxyalkyl, (C₃ - C₆)Alkoxycarbonylalkyl, (C₆ - C₈)Alkenyloxycarbonylalkyl oder (C₆ - C₈)Alkinyloxycarbonyl steht.

5. Verfahren zur Herstellung von anellierten (Oxa)-hydantoinen der Formel I, gemäß Anspruch 1,
**dadurch gekennzeichnet,** daß
a) eine Verbindung der Formel II mit einer Verbindung der Formel III 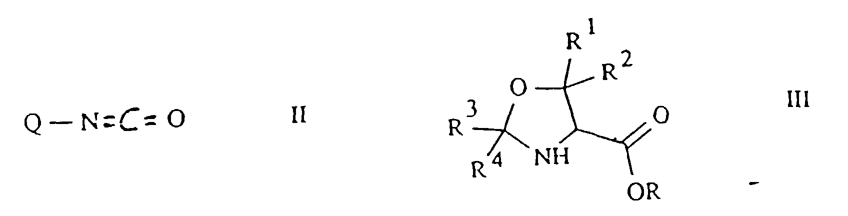 worin R = H, (C₁ - C₄)Alkyl oder Aktivester, gegebenenfalls in Gegenwart eines Säureakzeptors und gegebenenfalls in Gegenwart eines Verdünnungsmittels zur Reaktion gebracht wird, oder
b) eine Verbindung der Formel IV mit einer Verbindung der Formel V, gegebenenfalls In Gegenwart eines Säureakzeptors und gegebenenfalls in Gegenwart eines Verdünnungsmittels zur Reaktion gebracht wird, und das ggf. intermediär entstehende Reaktionsprodukt (III) mit einer Verbindung der Formel II ggf. in Gegenwart eines Säureakzeptors und ggf. in Gegenwart eines Verdünnungsmittels umgesetzt wird, wobei eine Verbindung der Formel VI entsteht, in der R = H ist, und 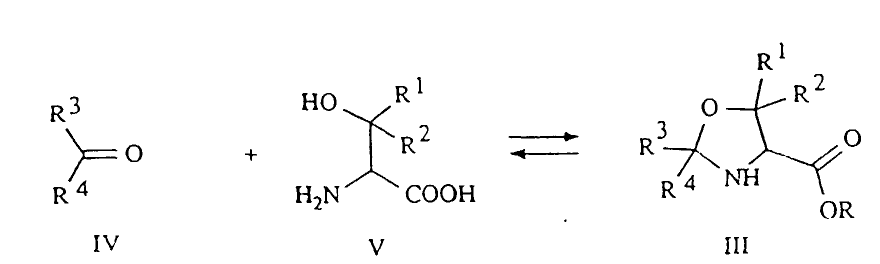 die dann unter Ringschluß in eine Verbindung der Formel I umgewandelt wird, oder
c) eine Verbindung der Formel III, in welcher R = H oder (C₁ - C₄)Alkyl ist, mit Phosgen oder einem Phosgenersatz zunächst in eine Verbindung der Formel VII und diese dann mit einer Verbindung der Formel VIII in eine Verbindung der Formel VI umgewandelt wird, 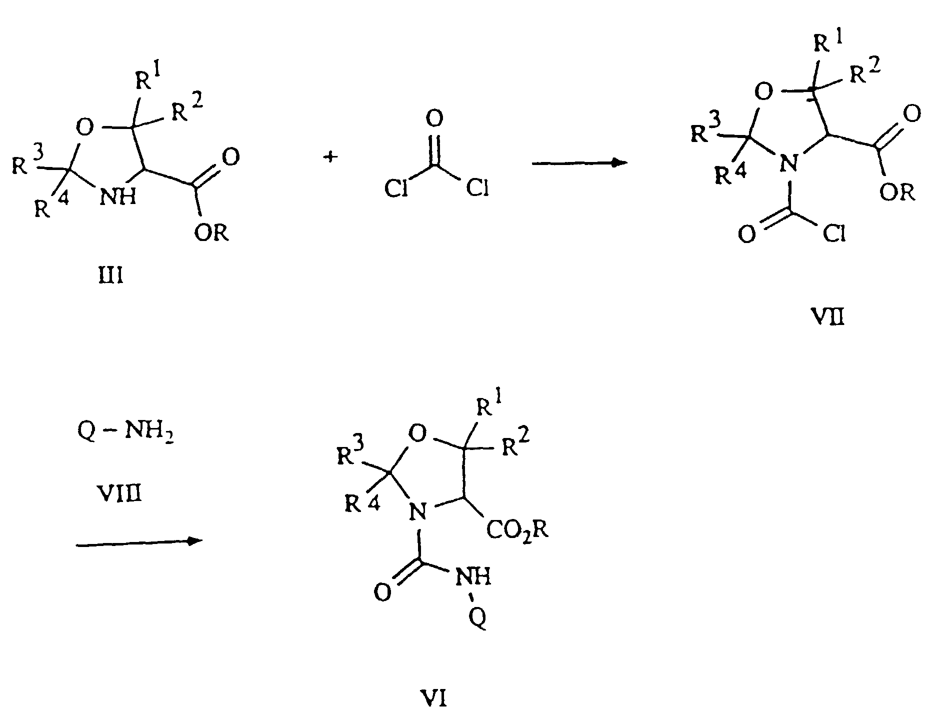 aus der unter Ringschluß eine Verbindung der Formel I erhalten wird, oder
d) eine Verbindung der Formel II mit einer Verbindung der Formel IX, gegebenenfalls in Gegenwart eines Verdünnungsmittels, zur Reaktion gebracht wird, wodurch eine Verbindung der Formel X 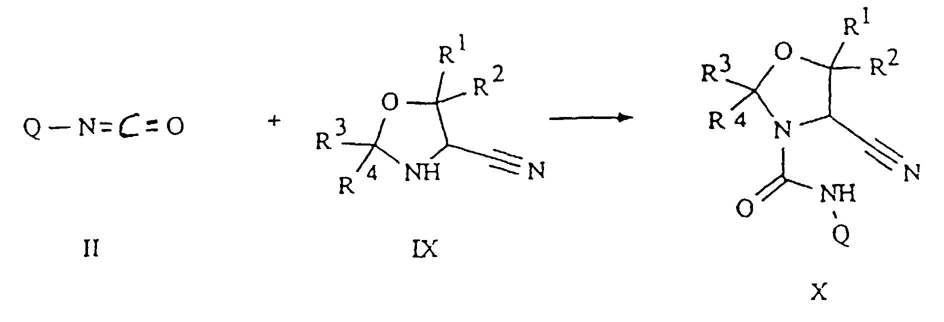 erhalten wird, die dann hydrolysiert und unter Ringschluß in eine Verbindung der Formel I umgewandelt wird, oder
e) eine Verbindung der Formel XI 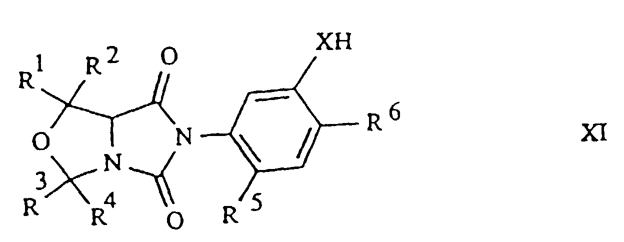 wobei X = O, S, NH ist,
mit einer Verbindung der Formel XII, XIII oder XIV 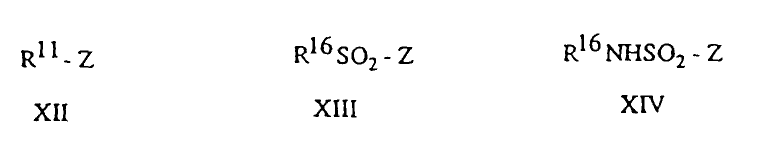 zur Reaktion gebracht werden, worin Z ein Chlor-, Brom- oder Jodatom ist, so daß eine Verbindung der Formel I erhalten wird.

6. Herbizide Zusammensetzung,
**gekennzeichnet durch**
einen effektiven Gehalt an einer Verbindung der Formel I gemäß Anspruch 1 und einem Trägerstoff hierfür.

7. Verwendung von Verbindungen der Formel I als Herbizide.

8. Verfahren zur Schadpflanzenkontrolle,
**dadurch gekennzeichnet,**
daß auf die Schadpflanzen oder ihre Umgebung eine herbizid wirksame Menge einer Verbindung gemäß Anspruch 1 ausgebracht wird.

## Claims

1. Anellated (oxa)hydantoins corresponding to formula I, 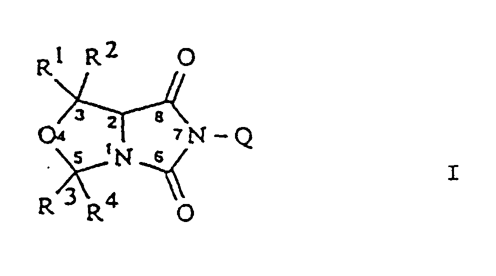 wherein R¹ and R², independently of one another, represent hydrogen or a group from among (C₁ - C₄)alkyl, (C₁ - C₂)haloalkyl or phenyl optionally fluorine-substituted,
R³ and R⁴, independently of one another, represent hydrogen, (C₁ - C₄)alkyl, phenyl, both optionally fluorine- and/or chlorine-, bromine- or methyl-substituted, or (C₁ - C₄)alkoxy; or together form a carbocyclic ring which may optionally be (C₁ - C₄)alkyl-substituted,
Q represents one of the groups Q₁ - Q₇ 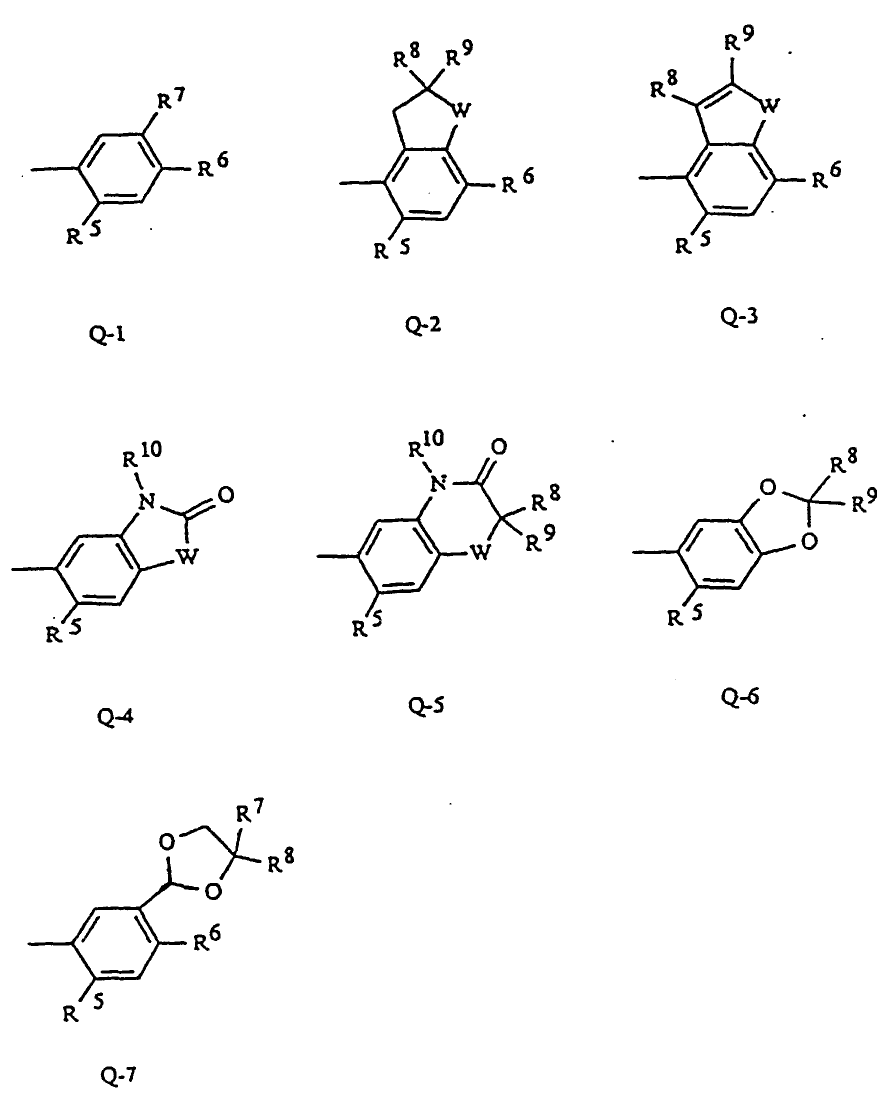 wherein
W represents O or S,
R⁵ represents hydrogen or halogen,
R⁶ represents (C₁ - C₂)alkyl, (C₁ - C₂)haloalkyl, OCH₃, SCH₃, OCHF₂, halogen, CN or NO₂,
R⁷ represents (C₁ - C₈)alkyl, (C₁ - C₈)haloalkyl, halogen, OR¹¹, S(O)ₙR¹¹, COR¹¹, CO₂R¹¹, C(O)SR¹¹, C(O)NR¹²R¹³, CHO, CH=CHCO₂R¹¹, CO₂N=CR¹⁴R¹⁵, NO₂, CN, NHSO₂R¹⁶ or NHSO₂NHR¹⁶,
R⁸ represents hydrogen, (C₁ - C₃)alkyl, (C₁ - C₃)haloalkyl or halogen,
R⁹ represents hydrogen, (C₁ - C₃)alkyl, (C₁ - C₃)haloalkyl or halogen; or if Q is Q-2 or Q-6, R⁸ and R⁹ together with the carbon atom to which they are bonded can be C=O;
R¹⁰ represents (C₁ - C₆)alkyl, (C₁ - C₆)haloalkyl, (C₂ - C₆)alkoxyalkyl, (C₃ - C₆)alkenyl or (C₃ - C₆)alkinyl,
R¹¹ represents (C₁ - C₈)alkyl, (C₃ - C₈)cycloalkyl, (C₃ - C₈)alkenyl, (C₃ - C₈)alkinyl, (C₁ - C₈)haloalkyl, (C₂ - C₈)alkoxyalkyl, (C₂ - C₈)alkylthioalkyl, (C₂ - C₈)alkylsulfinylalkyl, (C₂ - C₈)alkylsulfonylalkyl, (C₃ - C₈)alkoxyalkoxyalkyl, (C₄ - C₈)cycloalkylalkyl, (C₂ - C₄)carboxyalkyl, (C₃ - C₈)alkoxycarbonylalkyl, (C₆ - C₈)alkenyloxycarbonylalkyl, (C₆ - C₈)alkinyloxycarbonylalkyl, (C₄ - C₈)alkenoxyalkyl, (C₆ - C₈)cycloalkoxyalkyl, (C₄ - C₈)alkinyloxyalkyl, (C₃ - C₈)haloalkoxyalkyl, (C₄ - C₈)haloalkenyloxyalkyl, (C₄ - C₈)haloalkinyloxyalkyl, (C₆ - C₈)cycloalkylthioalkyl, (C₄ - C₈)alkenylthioalkyl, (C₄ - C₈)alkinylthioalkyl, (C₁ - C₄)alkyl substituted with phenoxy or benzyloxy, which may both optionally be halogen-, (C₁ - C₃)alkyl- or (C₁ - C₃)haloalkyl-substituted; (C₄ - C₈)trialkylsilylalkyl, (C₃ - C₈)cyanoalkyl, (C₃ - C₈)halocycloalkyl, (C₃ - C₈)haloalkenyl, (C₅ - C₈)alkoxyalkenyl, (C₅ - C₈)haloalkoxyalkenyl, (C₅ - C₈)alkylthioalkenyl, (C₃ - C₈)haloalkinyl, (C₅ - C₈)alkoxyalkinyl, (C₅ - C₈)haloalkoxyalkinyl, (C₅ - C₈)alkylthioalkinyl, (C₂ - C₈)alkylcarbonyl, benzyl optionally substituted with halogen, (C₁ - C₃)alkyl or (C₁ - C₃)haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷ C(O)NR¹²R¹³, CHR¹⁷C(O)NH₂, phenyl or pyridyl, both optionally substituted with halogen, (C₁ - C₃)alkyl, (C₁ - C₃)haloalkyl or (C₁ - C₄)alkoxy,
R¹² and R¹⁴, independently of one another, represent hydrogen or (C₁ - C₄)alkyl,
R¹³ and R¹⁵, independently of one another, represent (C₁ - C₄)alkyl, phenyl, optionally substituted with halogen, (C₁ - C₃)alkyl, (C₁ - C₃)haloalkyl or (C₁ - C₄)alkoxy, or
R¹² and R¹³ as -(CH₂)₅-, -(CH₂)₄-, or -CH₂CH₂OCH₂CH₂- can be combined to form rings, with one or more H atoms in each ring optionally being substitutable by (C₁ - C₃)alkyl, phenyl or benzyl;
R¹⁴ and R¹⁵ together with the carbon atom to which they are bonded can form a (C₃ - C₈)cycloalkyl group,
R¹⁶ represents (C₁ - C₄)alkyl or (C₁ - C₄)haloalkyl,
R¹⁷ represents hydrogen or (C₁ - C₃)alkyl,
R¹⁸ represents (C₁ - C₆)alkyl, (C₃ - C₆)alkenyl or (C₃ - C₆)alkinyl and
n represents 0, 1, 2.

2. Anellated (oxa)hydantoins corresponding to formula I according to claim 1,
characterised in that
at least one of the groups
R¹ and R², independently of one another, represent hydrogen or a group from among (C₁ - C₄)alkyl, (C₁ - C₂)haloalkyl or phenyl, which is optionally fluorine-substituted,
R³ and R⁴, independently of one another, represent hydrogen, (C₁ - C₃)alkyl, phenyl, optionally fluorine-substituted and/or chlorine-, bromine-, methyl-substituted, (C₁ - C₂)alkoxy, or together form a carbocyclic ring which may optionally be (C₁ - C₂)alkyl-substituted,
W represents O or S,
n represents 0, 1, 2,
R⁵ represents hydrogen or halogen,
R⁶ represents halogen or CN,
R⁷ represents (C₁ - C₄)alkyl, (C₁ - C₄)haloalkyl, halogen, OR¹¹, S(O)ₙR¹¹, COR¹¹, CO₂R¹¹, C(O)SR¹¹, C(O)NR¹²R¹³, CH=CHCO₂R¹¹, CO₂N=CR¹⁴R¹⁵, NHSO₂R¹⁶ or NHSO₂NHR¹⁶,
R⁸ represents hydrogen, (C₁ - C₃)alkyl or (C₁ - C₃)haloalkyl,
R⁹ represents hydrogen, (C₁ - C₃)alkyl or (C₁ - C₃)haloalkyl; or if Q is Q-2 or Q-6, R⁸ and R⁹ together with the hydrocarbon atom to which they are bonded can be C=O;
R¹⁰ represents (C₁ - C₄)alkyl, (C₁ - C₄)haloalkyl, (C₂ - C₄)alkoxyalkyl, (C₃ - C₆)alkenyl or (C₃ - C₆)alkinyl,
R¹¹ represents (C₁ - C₄)alkyl, (C₃ - C₆)cycloalkyl, (C₃ - C₆)alkenyl, (C₃ - C₆)alkinyl, (C₁ - C₄)haloalkyl, (C₂ - C₄)alkoxyalkyl, (C₂ - C₄)alkylthioalkyl, (C₂ - C₄)alkylsulfinylalkyl, (C₂ - C₄)alkylsulfonylalkyl, (C₃ - C₆)alkoxyalkoxyalkyl, (C₄ - C₈)cycloalkylalkyl, (C₂ - C₄)carboxyalkyl, (C₃ - C₆)alkoxycarbonylalkyl, (C₆ - C₈)alkenyloxycarbonylalkyl, (C₆ - C₈)alkinyloxycarbonylalkyl, (C₄ - C₆)alkenoxyalkyl, (C₆ - C₈)cycloalkoxyalkyl, (C₄ - C₆)alkinyloxyalkyl, (C₃ - C₆)haloalkoxyalkyl, (C₄ - C₈)haloalkenyloxyalkyl, (C₄ - C₆)haloalkinyloxyalkyl, (C₆ - C₈)cycloalkylthioalkyl, (C₄ - C₆)alkenylthioalkyl, (C₄ - C₆)alkinylthioalkyl, (C₁ - C₂)alkyl substituted with phenoxy or benzyloxy, which may both optionally be substituted with halogen, (C₁ - C₃)alkyl or (C₁ - C₃)haloalkyl; (C₄ - C₈)trialkylsilylalkyl, (C₃ - C₄)cyanoalkyl, (C₃ - C₆)halocycloalkyl, (C₃ - C₆)haloalkenyl, (C₅ - C₆)haloalkoxyalkenyl, (C₅ - C₆)alkylthioalkenyl, (C₃ - C₆)haloalkinyl, (C₅ - C₆)alkoxyalkinyl, (C₅ - C₆)haloalkoxyalkinyl, (C₅ - C₆)alkylthioalkinyl, (C₂ - C₄)alkylcarbonyl, benzyl optionally substituted with halogen, (C₁ - C₂)alkyl or (C₁ - C₂)haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷ C(O)NR¹²R¹³, CHR¹⁷C(O)NH₂, phenyl or pyridyl, both optionally substituted with halogen, (C₁ - C₃)alkyl, (C₁ - C₃)haloalkyl or (C₁ - C₄)alkoxy,
R¹² and R¹⁴, independently of one another, represent hydrogen or (C₁ - C₂)alkyl,
R¹³ and R¹⁵, independently of one another, represent (C₁ - C₂)alkyl, phenyl optionally substituted with halogen, (C₁ - C₂)alkyl, (C₁ - C₂)haloalkyl or (C₁ - C₃)alkoxy, or
R¹² and R¹³ as -(CH₂)₅-, -(CH₂)₄-, or -CH₂CH₂OCH₂CH₂- can be combined to form rings, with one or more H atoms in each ring optionally being substitutable by (C₁ - C₂)alkyl, phenyl or benzyl;
R¹⁴ and R¹⁵ together with the carbon atom to which they are bonded can form a (C₃ - C₆)cycloalkyl group,
R¹⁶ represents (C₁ - C₄)alkyl or (C₁ - C₄)haloalkyl,
R¹⁷ represents hydrogen or (C₁ - C₃)alkyl,
R¹⁸ represents (C₁ - C₄)alkyl, (C₃ - C₄)alkenyl or (C₃ - C₄)alkinyl.

3. Anellated (oxa)hydantoins corresponding to formula I according to claim 1, characterised in that at least one of the groups
R¹ and R², independently of one another, represent hydrogen or a group from among (C₁ - C₃)alkyl, (C₁ - C₂)haloalkyl or phenyl, which is optionally fluorine-substituted,
R³ and R⁴, independently of one another, represent hydrogen, (C₁ - C₃)alkyl, or also together form a 5- to 6-membered carbocyclic ring, which may optionally be (C₁ - C₄)alkyl-substituted,
W represents O or S,
R⁵ represents hydrogen, fluorine or chlorine,
R⁶ represents chlorine, bromine or cyanogen,
R⁷ represents OR¹¹ or CO₂R¹¹,
R⁸ and R⁹, independently of one another, represent hydrogen, (C₁ - C₂)alkyl or (C₁ - C₂)haloalkyl,
R¹⁰ represents (C₁ - C₂)alkyl, (C₁ - C₂)haloalkyl, (C₃ - C₄)alkenyl or (C₃ - C₄)alkinyl,
R¹¹ represents (C₁ - C₁)alkyl, (C₃ - C₆)cycloalkyl, (C₃ - C₆)alkenyl, (C₃ - C₆)alkinyl, (C₁ - C₄)haloalkyl, (C₂ - C₄)alkoxyalkyl, (C₂ - C₄)alkylthioalkyl, (C₂ - C₄)alkylsulfinylalkyl, (C₂ - C₄)alkylsulfonylalkyl, (C₃ - C₆)alkoxyalkoxyalkyl, (C₄ - C₈)cycloalkylalkyl, (C₂ - C₄)carboxyalkyl, (C₃ - C₆)alkoxycarbonylalkyl, (C₆ - C₈)alkenyloxycarbonylalkyl, (C₆ - C₈)alkinyloxycarbonylalkyl, (C₆ - C₈)cycloalkoxyalkyl, (C₄ - C₆)alkenyloxyalkyl, (C₄ - C₆)alkinyloxyalkyl, (C₃ - C₆)haloalkoxyalkyl, (C₄ - C₈)haloalkenyloxyalkyl, (C₄ - C₆)haloalkinyloxyalkyl, (C₆ - C₈)cycloalkylthioalkyl, (C₄ - C₆)alkenylthioalkyl, (C₄ - C₆)alkinylthioalkyl, (C₁ - C₂)alkyl substituted with phenoxy or benzyloxy, which are both optionally substituted with halogen, (C₁ - C₃)alkyl or (C₁ - C₃)haloalkyl; (C₄ - C₈)trialkylsilylalkyl, (C₃ - C₄)cyanoalkyl, (C₃ - C₆)halocycloalkyl, (C₃ - C₆)haloalkenyl, (C₅ - C₆)alkoxyalkenyl, (C₅ - C₆)haloalkoxyalkenyl, (C₅ - C₆)alkylthioalkenyl, (C₃ - C₆)haloalkinyl, (C₅ - C₆)alkoxyalkinyl, (C₅ - C₆)haloalkoxyalkinyl, (C₅ - C₆)alkylthioalkinyl, (C₂ - C₄)alkylcarbonyl, benzyl optionally substituted with halogen, (C₁ - C₂)alkyl or (C₁ - C₂)haloalkyl; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, phenyl or pyridyl, both optionally substituted with fluorine, chlorine, bromine, (C₁ - C₂)haloalkyl or (C₁ - C₂)alkoxy,
R¹² represents hydrogen or (C₁ - C₂)alkyl,
R¹³ represents (C₁ - C₂)alkyl, phenyl optionally substituted with fluorine, chlorine, bromine, (C₁ - C₂)alkyl, (C₁ - C₂)haloalkyl or (C₁ - C₂)alkoxy, or
R¹² and R¹³ as -(CH₂)₅-, -(CH₂)₄-, or -CH₂CH₂OCH₂CH₂- can be combined to form rings, with one or more H atoms in each ring optionally being substitutable by (C₁ - C₂)alkyl;
R¹⁷ represents hydrogen or (C₁ - C₂)alkyl,
R¹⁸ represents (C₁ - C₂)alkyl, (C₃ - C₄)alkenyl or (C₃ - C₄)alkinyl.

4. Anellated (oxa)hydantoins corresponding to formula I according to claim 1,
characterised in that
at least one of the groups
R¹ and R², independently of one another, represent hydrogen, (C₁ - C₃)alkyl, (C₁ - C₂)haloalkyl or phenyl,
R³ and R⁴, independently of one another, represent hydrogen or (C₁ - C₃)alkyl, or also together can form a 5- to 6-membered carbocyclic ring,
R⁵ represents fluorine or chlorine,
R⁶ represents chlorine,
R⁷ represents OR¹¹ or CO₂R¹¹,
R¹² represents (C₁ - C₄)alkyl, (C₃ - C₆)cycloalkyl, (C₃ - C₆)alkenyl, (C₃ - C₄)alkinyl, (C₁ - C₃)haloalkyl, (C₂ - C₄)alkoxyalkyl, (C₃ - C₆)alkoxycarbonylalkyl, (C₆ - C₈)alkenyloxycarbonylalkyl or (C₆ - C₈)alkinyloxycarbonyl.

5. Method for the preparation of anellated (oxa)hydantoins corresponding to formula I, according to claim 1,
characterised in that
(a) a compound corresponding to formula II is caused to react with a compound corresponding to formula III 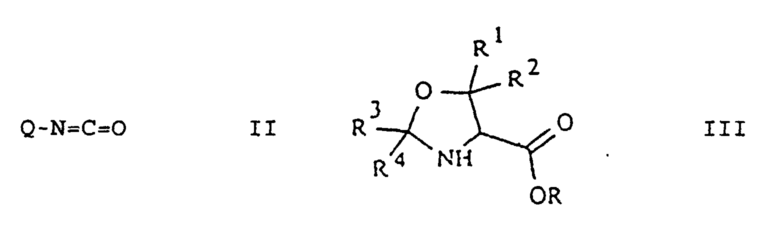 wherein R stands for H, (C₁ - C₄)alkyl or active ester, optionally in the presence of an acid acceptor and optionally in the presence of a diluent, or
b) a compound corresponding to formula IV is caused to react with a compound corresponding to formula V, optionally in the presence of an acid acceptor and optionally in the presence of a diluent, and the reaction product (III) possibly arising as an intermediate is reacted with a compound corresponding to formula II optionally in the presence of an acid acceptor and optionally in the presence of a diluent, with the formation of a compound corresponding to formula VI, wherein R is H, and 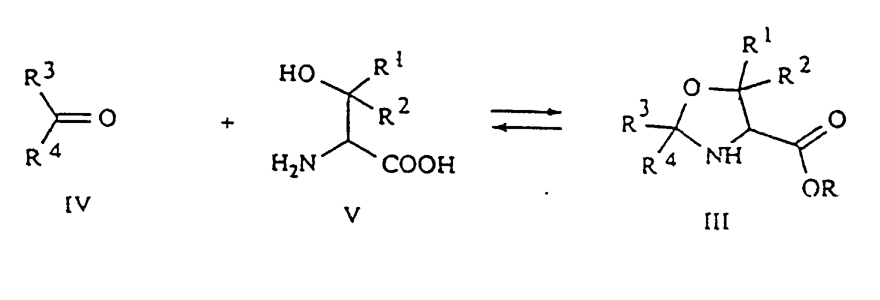 this is then converted, with ring closure, into a compound corresponding to formula I, or
c) a compound corresponding to formula III, wherein R is H or (C₁ - C₄)alkyl, together with phosgene or a phosgene substitute, is first of all converted into a compound corresponding to formula VII and then this, together with a compound corresponding to formula VIII, is converted to a compound corresponding to formula VI, 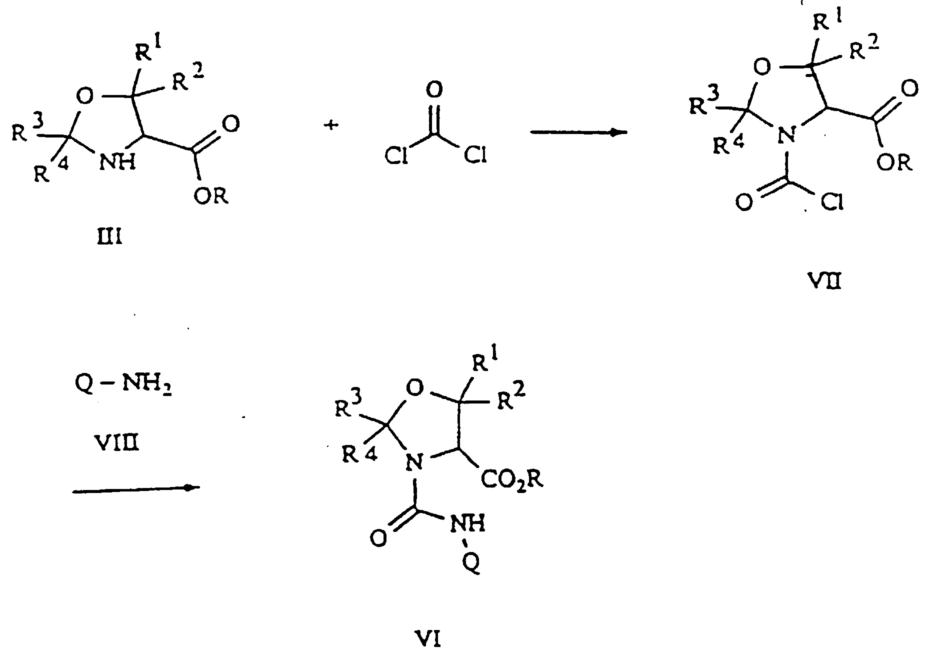 from which, with ring closure, a compound corresponding to formula I is obtained, or
d) a compound corresponding to formula II is caused to react with a compound corresponding to formula IX, optionally in the presence of a diluent, whereby a compound corresponding to formula X 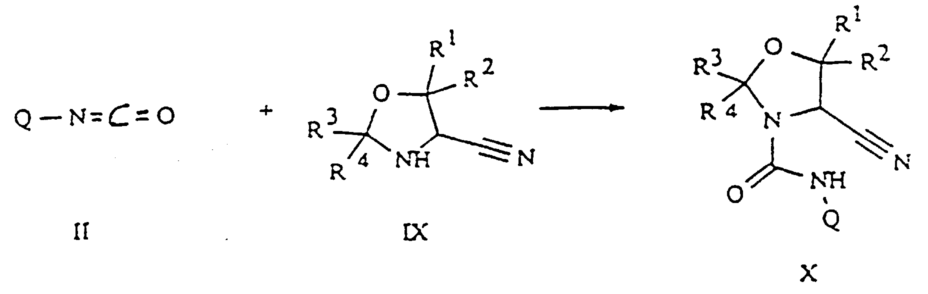 is obtained, which is then hydrolysed and converted, with ring closure, to a compound corresponding to formula I, or
e) a compound corresponding to formula XI 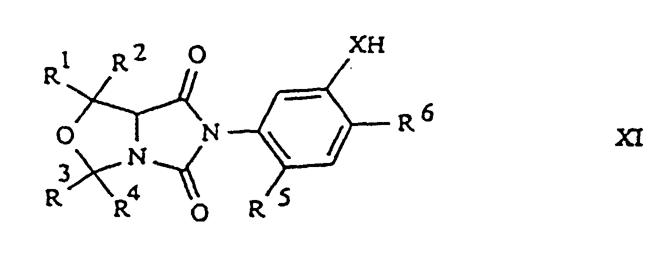 wherein X is O, S, NH,
is caused to react with a compound corresponding to formula XII, XIII or XIV 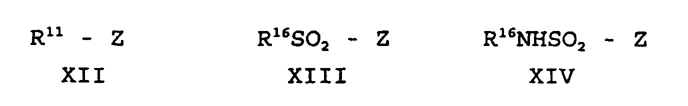 wherein Z is an atom of chlorine, bromine or iodine, so that a compound corresponding to formula I is obtained.

6. Herbicidal composition, characterised by having an effective content of a compound corresponding to formula I according to claim 1, and a carrier for this.

7. Use of compounds corresponding to formula I as herbicides.

8. Process for the control of noxious plants, characterised in that a herbicidally active quantity of a compound according to claim 1 is applied to the noxious plants or their surroundings.

## Revendications

1. (Oxa)hydantoïnes condensées de formule I : 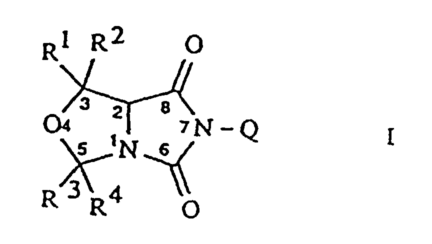 dans laquelle :
- R¹ et R² indépendamment l'un de l'autre, représentent de l'hydrogène ou un reste choisi dans la série des alkyles en C₁-C₄, des haloalkyles en C₁-C₂ ou du phényle éventuellement substitué par du fluor,
- R³ et R⁴ indépendamment l'un de l'autre, représentent de l'hydrogène, un alkyle en C₁-C₄ un phényle, les deux éventuellement substitués par du fluor et/ou du chlore, du brome ou un méthyle, ou un alcoxy en C₁-C₄, ou bien forment ensemble un noyau carbocyclique qui peut être substitué éventuellement par un alkyle en C₁-C₄,
- Q représente un des radicaux Q₁ à Q₇ 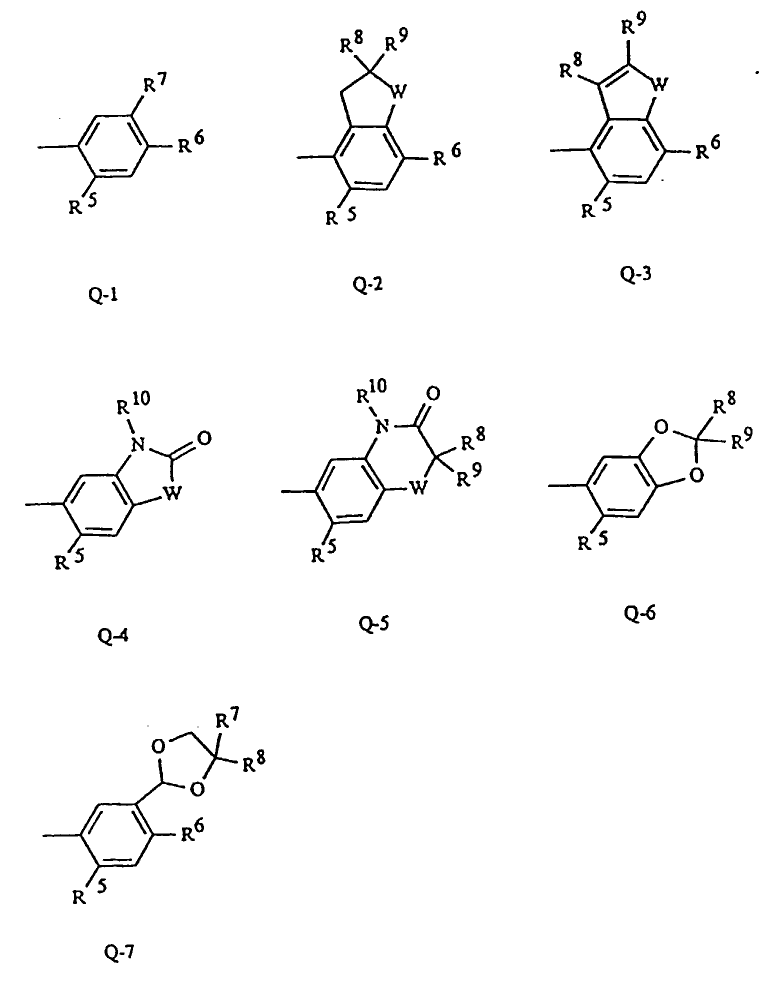
dans lesquels :
- W représente O ou S,
- R⁵ représente de l'hydrogène ou un halogène,
- R⁶ représente un alkyle en C₁-C₂, un haloalkyle en C₁-C₂, OCH₃, SCH₃, OCHF₂, halogène, CN ou NO₂,
- R⁷ représente un alkyle en C₁-C₈, un haloalkyle en C₁-C₈, un halogène, OR¹¹, S(O)ₙR¹¹, COR¹¹, CO₂R¹¹, C(O)SR¹¹, C(O)NR¹²R¹³, CHO, CH = CHCO₂R¹¹, CO₂N = CR¹⁴R¹⁵ , NO₂, CN, NHSO₂R¹⁶ ou NHSO₂NHR¹⁶,
- R⁸ représente de l'hydrogène, un alkyle en C₁-C₃, ou un haloalkyle en C₁-C₃, ou un halogène,
- R⁹ représente de l'hydrogène, un alkyle en C₁-C₃, ou un haloalkyle en C₁-C₃, ou un halogène, ou lorsque Q = Q-2 ou Q-6, R⁸ et R⁹ peuvent conjointement avec l'atome de carbone auquel ils sont reliés, être C=O,
- R¹⁰ représente un alkyle en C₁-C₆, un haloalkyle en C₁-C₆, un alkoxyalkyle en C₂-C₆, un alkényle en C₃-C₆ ou un alkinyle en C₃-C₆,
- R¹¹ représente un alkyle en C₁-C₈, un cycloalkyle en C₃-C₈, un alkényle en C₃-C₈, un alkinyle en C₃-C₈, un haloalkyle en C₁-C₈, un alkoxyalkyle en C₂-C₈, un alkylthioalkyle en C₂-C₈, un alkyle en C₂-C₈ sulfinylalkyle, un alkyle en C₂-C₈ sulfonylalkyle, un alkoxyalkoxyalkyle en C₃-C₈, un cycloalkylalkyle en C₄-C₈, un carboxyalkyle en C₂-C₄, un alkoxycarbonylalkyle en C₃-C₈, un alkényloxycarbonylalkyle en C₆-C₈, un alkinyloxycarbonylalkyle en C₆-C₈, un alkénoxyalkyle en C₄-C₈, un cycloalkoxyalkyle en C₆-C₈, un alkinyloxyalkyle en C₄-C₈, un haloalkoxyalkyle en C₃-C₈, un haloalkényloxyalkyle en C₄-C₈, un haloalkinyloxyalkyle en C₄-C₈, un cycloalkylthioalkyle en C₆-C₈, un alkénylthioalkyle en C₄-C₈, un alkinylthioalkyle en C₄-C₈, un alkyle en C₁-C₄ substitué par un phénoxy, ou un benzyloxy, tous les deux pouvant être substitués éventuellement par un halogène, un alkyle en C₁-C₃, ou par un haloalkyle en C₁-C₃ ; un (trialkyle en C₄-C₈) silylalkyle, un cyanoalkyle en C₃-C₈, un halocycloalkyle en C₃-C₈, un haloalkényle en C₃-C₈, un alkoxyalkényle en C₅-C₈ un haloalcoxyalkényle en C₅-C₈, un alkyle en C₅-C₈ thioalkényle, un haloalkinyle en C₃-C₈, un alcoxyalkynyle en C₅-C₈, un haloalcoxyalkinyle en C₅-C₈, un alkyle en C₅-C₈ thioalkinyl, un alkyle en C₂-C₈ carbonyle, un benzyle éventuellement substitué par un halogène, un alkyle en C₁-C₃ ou un haloalkyle en C₁-C₃, CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, phényle ou pyridyle, tous les deux éventuellement substitués par un halogène, un alkyle en C₁-C₃, un haloalkyle en C₁-C₃, ou un alcoxy en C₁-C₄,
- R¹² et R¹⁴ indépendamment l'un de l'autre, représentent de l'hydrogène ou un alkyle an C₁-C₄,
- R¹³ et R¹⁵ indépendamment l'un de l'autre représentent un alkyle en C₁-C₄, un phényle éventuellement substitué par un halogène, un alkyle en C₁-C₃, un haloalkyle en C₁-C₃ ou un alcoxy an C₁-C₄, ou
- R¹² et R¹³ peuvent être combinés en tant que (CH₂)₅, (CH₂)₄ ou CH₂CH₂OCH₂CH₃, en des cycles dans lesquels un ou plusieurs atomes d'hydrogène dans chaque cycle peuvent être éventuellement substitués par un alkyle en C₁-C₃, un phényle ou un benzyle,
- R¹⁴ et R¹⁵ ensemble avec l'atome de carbone, auquel ils sont reliés, peuvent former un groupe cycloalkyle en C₃-C₈,
- R¹⁶ représente un alkyle en C₁-C₄, ou un haloalkyle en C₁-C₄,
- R¹⁷ représente de l'hydrogène ou un alkyle en C₁-C₃,
- R¹⁸ représente un alkyle en C₁-C₆, un alkényle en C₃-C₆ ou un alkinyle en C₃-C₆ et
- n représente 0, 1 ou 2.

2. (Oxa)hydantoïnes condensées de formule I selon la revendication 1,
caractérisées en ce qu'
au moins un des radicaux
- R¹ et R² indépendamment l'un de l'autre représentent de l'hydrogène ou un groupe de la série alkyle en C₁-C₄, haloalkyle en C₁-C₂ ou phényle qui peut être éventuellement substitué par du fluor,
- R³ et R⁴ indépendamment l'un de l'autre, représentent de l'hydrogène, un alkyle en C₁-C₃, ou un phényle éventuellemnt substitué par du fluor et/ou un alkoxy et C₁-C₂ substitué par du chlore, du brome ou un méthyle ; ou bien ils forment ensemble un cycle carbocyclique qui peut éventuellement être substitué par un alkyle en C₁-C₂,
- W représente O ou S,
- n représente 0, 1, 2,vnb
- R⁵ représente de l'hydrogène ou un halogène,
- R⁶ représente un halogène ou CN,
- R⁷ représente un alkyle en C₁-C₄, un haloalkyle en C₁-C₄, un halogène, OR¹¹, S(O)ₙR¹¹, COR¹¹, CO₂R¹¹, C(O)SR¹¹, C(O)NR¹²R¹³, CH=CHCO₂R¹¹, CO₂N=CR¹⁴R¹⁵, NHSO₂R¹⁶ ou NHSO₂NHR¹⁶,
- R⁸ représente de l'hydrogène, un alkyle en C₁-C₃ ou un haloalkyle en C₁-C₃,
- R⁹ représente de l'hydrogène, un alkyle en C₁-C₃ ou un haloalkyle en C₁-C₃, ou quand Q est Q-2 ou Q-6, R⁸ et R⁹ peuvent être C=0 ensemble avec l'atome d'hydrocarbure auquel il sont reliés,
- R¹⁰ représente un alkyle en C₁-C₆, un haloalkyle en C₁-C₆, un alkoxyalkyle en C₂-C₆, un alkényle en C₃-C6 ou un alkynyle en C₃-C₆,
- R¹¹ représente un alkyle en C₁-C₄, un cycloalkyle en C₃-C₆, un alkényle en C₃-C₆, un alkinyle en C₃-C₆, un haloalkyle en C₁-C₄, un alkoxyalkyle en C₂-C₄, un alkylthioalkyle en C₂-C₄, un alkylsulfinylakyle en C₂-C₄, un alkylsulfonylalkyle en C₂-C₄, un alkoxyalkoxyalkyle en C₃-C₆, un cycloalkylalkyle en C₄-C₈, un carboxyalkyle en C₂-C₄, un alkoxycarbonylalkyle en C₃-C₆, un alkényloxycarbonylalkyle en C₆-C₈, un alkényloxycarbonylalkyle en C₆-C₈, un alkénoxyalkyle en C₄-C₆, un cycloalkoxyalkyle en C₆-C₈, un alkinyloxyalkyle en C₄-C₆, un haloalkoxyalkyle en C₃-C₆, un haloalkényloxyalkyle en C₄-C₈, un haloalkinyloxyalkyle en C₄-C₆, un cycloalkylthioalkyle en C₆-C₈, un alkénylthioalkyle en C₄-C₆, un alkinylthioalkyle en C₄-C₆, un alkyle en C₁-C₂ substitué par un phénoxy ou un benzyloxy, tous les deux pouvant être substitués par un halogène, un alkyle en C₁-C₃ ou par un haloalkyle en C₁-C₃ ; un trialkylsilylalkyle en C₄-C₈, un cyanoalkyle en C₃-C₄, un halocycloalkyle en C₃-C₆, un haloalkényle en C₃-C₆, un haloalcoxyalkényle en C₅-C₆, un alkylthioalkényle en C₅-C₆, un haloalkinyle en C₃-C₆, un alkoxyalkinyle en C₅-C₆, un haloalkoxyalkinyle en C₅-C₆, un alkylthioalkinyle en C₅-C₆, un alkylcarbonyle en C₂-C₄ un benzyle éventuellement substitué par un halogène, un alkyle en C₁-C₂ ou un haloalkyle en C₁-C₂ ; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, un phényle ou un pyridyle, tous les deux éventuellement substitués par un halogène, un alkyle en C₁-C₃, un haloalkyle en C₁-C₃ ou un alkoxy en C₁-C₄,
- R¹² et R¹⁴, indépendamment l'un de l'autre représentent de l'hydrogène ou un alkyle en C₁-C₂,
- R¹³ et R¹⁵ indépendamment l'un de l'autre, représentent un alkyle en C₁-C₂, un phényle éventuellement substitué par un halogène, un alkyle en C₁-C₂, un haloalkyle en C₁-C₂ ou par un alkoxy en C₁-C₂, ou
- R¹² et R¹³ sous forme de - (CH₂)₅-, -(CH₂)₄- ou -CH₂CH₂OCH₂CH₂-peuvent être combinés en cycles dans lesquels un ou plusieurs atomes d'H dans chaque cycle peuvent être éventuellement substitués par un alkyle en C₁-C₂, un phényle ou un benzyle,
- R¹⁴ et R¹⁵ conjointement avec le carbone auxquels ils sont reliés peuvent former un groupe cycloalkyle en C₃-C₆
- R¹⁶ représente un alkyle en C₁-C₄ ou un haloalkyle en C₁-C₄
- R¹⁷ représente de l'hydrogène ou un alkyle en C₁-C₃,
- R¹⁸ représente un alkyle en C₁-C₄, un alkényle en C₃-C₄ ou un alkinyle en C₃-C₄.

3. (Oxa)hydantoïnes condensées de formule I selon la revendication 1,
caractérisé en ce qu'
au moins un des radicaux :
- R¹ et R² indépendamment l'un de l'autre, représentent de l'hydrogène ou un reste de la série alkyle en C1-C3, haloalkyle en C1-C2 ou phényle, qui est éventuellement substitué par du fluor,
- R³ et R⁴, indépendamment l'un de l'autre, représentent de l'hydrogène, un alkyle en C1-C3, ou aussi ensemble forment un composé carbocyclique à 5 ou 6 maillons qui peut être substitués le cas échéant, par un alkyle en C1-C4,
- W représente O ou S
- R⁵ représente de l'hydrogène ,le fluor ou le chlore,
- R⁶ représente le chlore, le brome ou le cyano,
- R⁷ représente l'hydrogène, OR¹¹ ou CO₂R¹¹,
- R⁸ et R⁹ indépendamment l'un de l'autre, représentent l'hydrogène, un alkyle en C₁-C₂ ou un haloalkyle en C₁-C₂,
- R¹⁰ représente un alkyle en C₁-C₂, un haloalkyle en C₁-C₂, un alkényle en C₃-C₄, ou un alkinyle en C₃-C₄,
- R¹¹ représente un alkyle en C₁-C₄, un cycloalkyle en C₃-C₆, un alkényle en C₃-C₆, un alkinyle en C₃-C₆, un haloalkyle en C₁-C₄, un alkoxyalkyle en C₂-C₄, un alkylthioalkyle en C₂-C₄, un alkylsulfinylalkyle en C₂-C₄, un alkylsulfonylalkyle en C₂-C₄, un alkoxyalkoxyalkyle en C₃-C₆, un cycloalkylalkyle en C₄-C₈, un carboxyalkyle en C₂-C₄, un alkoxycarbonylalkyle en C₃-C₆, un alkényloxycarbonylalkyle en C₆-C₈, un alkinyloxycarbonylalkyle en C₆-C₈, un cycloalkoxyalkyle en C₆-C₈, un alkényloxyalkyle en C₄-C₆, un alkinyloxyalkyle en C₄-C₆, un haloaloxyalkyle en C₃-C₆, un haloalkényloxyalkyle en C₄-C₈, un haloalkinyloxyalkyle en C₄-C₆, un cycloalkylthioalkyle en C₆-C₈, un alkénylthioalkyle en C₄-C₆, un alkinylthioalkyle en C₄-C₆, un alkyle en C₁-C₂ substitué par un phénoxy ou un benzyloxy, tous les deux sont éventuellement substitués par un halogène, un alkyle en C₁-C₃ ou un haloalkyle en C₁-C₃ ; un trialkylsilylalkyle en C₄-C₈, un cyanoalkyle en C₃-C₄, un halocycloalkyle en C₃-C₆, un haloalkényle en C₃-C₆, un alkoxyalkényle en C₅-C₆, un haloalkoxyalkényle en C₅-C₆, un alkylthioalkényle en C₅-C₆, un haloalkinyle en C₃-C₆, un alkoxyalkinyle en C₅-C₆, un haloalkoxyalkinyle en C₅-C₆, un alkylthioalkinyle en C₅-C₆, un alkylcarbonyle en C₂-C₄, un benzyle éventuellement substitué par un halogène, un alkyle en C₁-C₂ ou par un haloalkyle en C₁-C₂ ; CHR¹⁷COR¹⁸, CHR¹⁷P(O)(OR¹⁸)₂, P(O)(OR¹⁸)₂, CHR¹⁷P(S)(OR¹⁸)₂, CHR¹⁷C(O)NR¹²R¹³, CHR¹⁷C(O)NH₂, phényle ou pyridyle, tous les deux le cas échéant, substitués par un fluor, un chlore, un brome, un haloalkyle en C₁-C₂ ou un alkoxy en C₁-C₂,
- R¹² représente de l'hydrogène ou un alkyle en C₁-C₂,
- R¹³ représente un alkyle en C₁-C₂, un phényle éventuellement substitué par un fluor, un chlore, un brome, un alkyle en C₁-C₂, un haloalkyle en C₁-C₂ ou un alkoxy en C₁-C₂, ou bien
- R¹² et R¹³ peuvent être combinés sous forme de -(CH₂)₅-, -(CH₂)₄- ou -CH₂CH₂OCH₂CH₂-, en des cycles dans lesquels un ou plusieurs atomes d'H dans chaque cycle peuvent être substitués éventuellement par un alkyle en C₁-C₂,
- R¹⁷ représente de l'hydrogène ou un alkyle en C₁-C₂,
- R¹⁸ représente un alkyle en C₁-C₂, un alkényle en C₃-C₄, ou un alkinyle en C₃-C₄,

4. (Oxa)hydantoïnes condensées de formule I selon la revendication 1,
caractérisé en ce qu'
au moins un des radicaux :
- R¹ et R² indépendamment l'un de l'autre représentent de l'hydrogène, un alkyle en C₁-C₃, un haloalkyle en C₁-C₂, ou un phényle,
- R³ et R⁴, indépendamment l'un de l'autre, représentent de l'hydrogène ou un alkyle en C₁-C₃, ou aussi peuvent former un cycle carbocyclique à 5 ou 6 membres,
- R⁵ représente du fluor ou du chlore,
- R⁶ représente du chlore,
- R⁷ représente OR¹¹ ou CO₂R¹¹,
- R¹¹ représente un alkyle en C₁-C₄, un cycloalkyle en C₃-C₆, un alkényle en C₃-C₆, un alkinyle en C₃-C₄, un haloalkyle en C₁-C₃, un alkoxyalkyle en C₂-C₄, un alkoxycarbonylalkyle en C₃-C₆, un alkényloxycarbonylalkyle en C₆-C₈ ou un alkinyloxycarbonyle en C₆-C₈.

5. Procédé de fabrication d'(oxa)hydantoïnes condensées de formule I selon la revendication 1,
caractérisé en ce que
a) on met en réaction un composé de formule II avec un composé de formule III 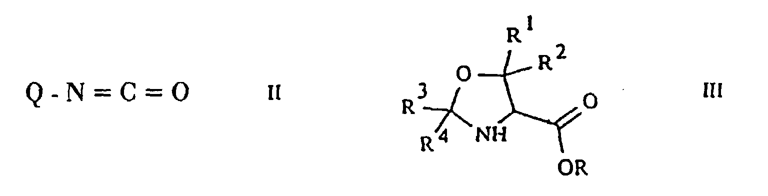 dans laquelle R=H, un alkyle en C₁-C₄, ou un ester réactif, le cas échéant en présence d'un accepteur d'acide et le cas échéant en présence d'un agent de dilution, ou
b) on met en réaction un composé de formule IV avec un composé de formule V, le cas échéant en présence d'un accepteur d'acide et le cas échéant en présence d'un agent de dilution et on fait réagir le produit réactionnel (III) formé le cas échéant intermédiairement, avec un composé de formule (II) éventuellement en présence d'un accepteur d'acide et éventuellement en présence d'un agent de dilution, dans laquelle un composé de formule VI se forme dans laquelle R est = à H et 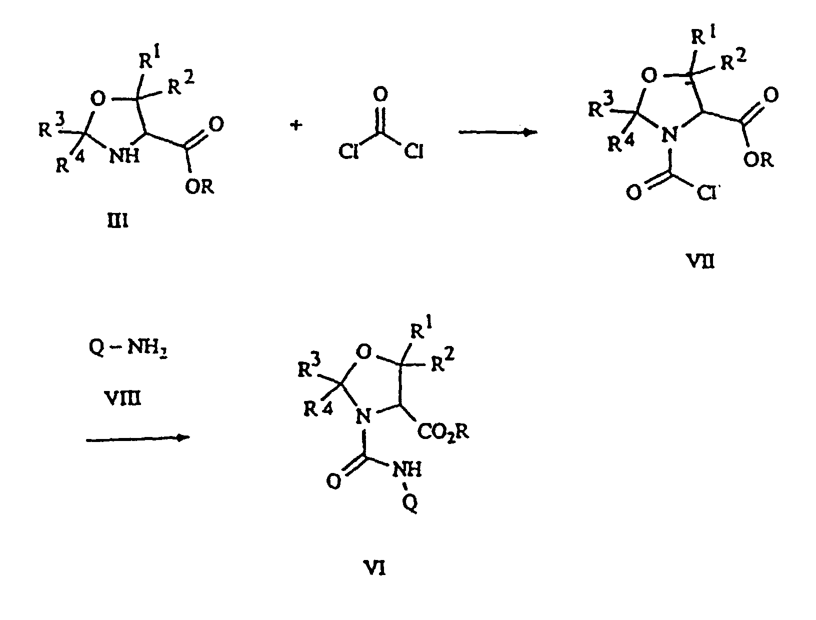 qui est transformé ensuite par fermeture du cycle en un composé de formule I, ou
c) on transforme un composé de formule III dans laquelle R est = à H ou à un alkyle en C₁-C₄ avec du phosgène ou avec un produit de substitution du phosgène, en premier lieu en un composé de formule VII et on convertit celui-ci ensuite avec un composé de formule VII en un composé de formule VI à partir duquel par fermeture du cycle, on obtient un composé de formule I, ou
d) on met en réaction un composé de formule II avec un composé de formule IX, éventuellement en présence d'un agent de dilution, grâce à quoi un composé de formule X 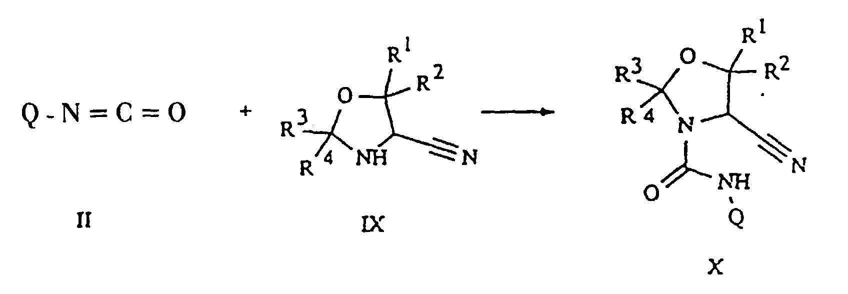 est obtenu qui est ensuite hydrolysé et converti par fermeture du cycle en un composé de formule I ou
e) on met en réaction un composé de formule XI 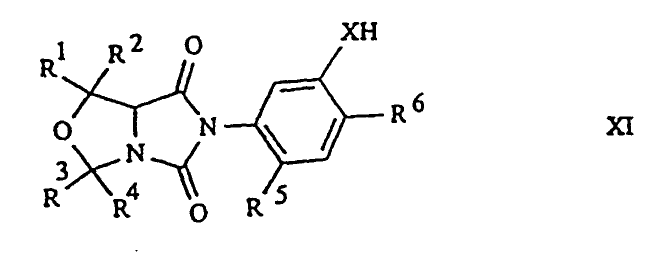 dans laquelle X est = à O, S ou NH
avec un composé de formule XII, de formule XIII ou de formule XIV 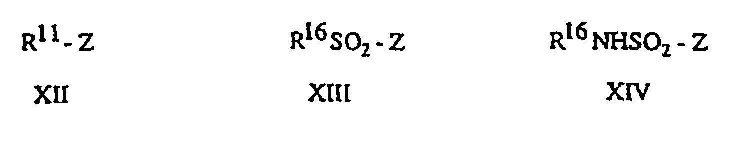 dans laquelle Z est un atome de chlore, de brome ou d'iode de sorte qu'on obtient un composé de formule I.

6. Composition herbicide,
caractérisée par
une teneur efficace en un composé de formule I conformément à la revendication 1, et par une substance de support dans ce but.

7. Utilisation des composés de formule I en tant qu'herbicides.

8. Procédé pour le contrôle des plantes nuisibles,
caractérisé en ce qu'
on applique sur les plantes nuisibles ou sur leur environnement, une quantité active comme herbicide, d'un composé conformément à la revendication 1.
